# EUROPÄISCHE PATENTSCHRIFT

(11) **EP 1 218 383 B1**
(45) Veröffentlichungstag und Bekanntmachung des Hinweises auf die Patenterteilung: **05.11.2008**
(21) Anmeldenummer: 00967693.3
(22) Anmeldetag: 18.09.2000
(51) Int. Cl.: C07D 487/04, C07D 513/04, A61K 31/41, A61P 25/00, A61P 43/00

(54) **BICYCLISCHE IMIDAZO-5-YL-AMINDERIVATE**
BICYCLIC IMIDAZO-5-YL-AMINE DERIVATIVES
DERIVES D'IMIDAZO-5-YL-AMINE BICYCLIQUES

(30) Priorität: 08.10.1999 DE 19948436; 08.10.1999 DE 19948434
(43) Veröffentlichungstag der Anmeldung: 03.07.2002
(73) Patentinhaber: Grünenthal GmbH, 52078 Aachen (DE)
(72) Erfinder: GERLACH, Matthias, 63636 Brachttal (DE); MAUL, Corinna, 52066 Aachen (DE)
(86) Internationale Anmeldenummer: PCT/EP2000/009097
(87) Internationale Veröffentlichungsnummer: WO 2001/027118

(56) Entgegenhaltungen:
- EP-A- 0 518 033
- PALAGIANO F ET AL: "Research on heterocyclic compounds. XXXIV. Synthesis and SAR study of some imidazo[2,1-b]thiazole carboxylic and acetic acids with antiinflammatory and analgesic activities" EUROPEAN JOURNAL OF MEDICINAL CHEMISTRY.CHIMICA THERAPEUTICA,FR,EDITIONS SCIENTIFIQUE ELSEVIER, PARIS, Bd. 30, Nr. 11, 1995, Seiten 901-909, XP004040219 ISSN: 0223-5234
- H.BIENAYME ET AL.: "A New Heterocyclic Multicomponent Reaction For the Combinatorial Synthesis of Fused 3-Aminoimidazoles." ANGEW. CHEM. INTL. EDN., Bd. 37, Nr. 16, 1998, Seiten 2234-2237, XP000978871 in der Anmeldung erwähnt

## Beschreibung

Die vorliegende Erfindung betrifft substituierte bicyclische Imidazo-5-yl-amine und Arzneimittel enthaltend diese Verbindungen.

Einzelne Vertreter aus der Klasse der bicyclischen Imidazo-5-yl-amine sind in der EP-A-0 518 033 beschrieben. Dabei handelt es sich stets um solche Verbindungen, die an dem nicht den anellierten Ringsystemen angehörenden Imidazol-Stickstoff einen über eine kurze Alkylbrücke gebundenen aromatischen Substituenten tragen. Die entsprechenden Verbindungen werden in der EP-A-0 518 033 als starke Angiotensin-Antagonisten beschrieben, die in Arzneimitteln zur Behandlung von Kreislauferkrankungen wie Bluthochdruck eingesetzt werden können.

In der Folge wurden Versuche unternommen, auch solche bicyclischen Imidazo-5-yl-amine herzustellen, die an dem nicht den anellierten Ringsystemen angehörenden Imidazol-Stickstoff nicht substituiert sind. Diese Versuche hatten jedoch keinen (K. Groebke et al., Synlett 1998, 661) oder nur geringen Erfolg (H. Bienayme, K. Bouzid, Angew. Chem. 1998, 110 (16), 2349).

Der vorliegenden Erfindung lag daher die Aufgabe zugrunde, bicyclischen Imidazo-5-yl-amine, die an dem nicht den anellierten Ringsystemen angehörenden Imidazol-Stickstoff nicht substituiert sind, und diese enthaltende Arzneimittel bereitzustellen.

Gegenstand der Erfindung sind daher bicyclische Imidazo-5-yl-amine der allgemeinen Formel I, 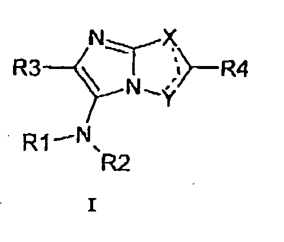 worin
R¹ C(CH₃)₃
   Phenyl, C₄-C₈-Cycloalkyl,
   1,1,3,3-Tetramethylbutyl oder CH₂R^{a}, wobei R^{a} für Wasserstoff, C₁-C₈-Alkyl (verzweigt oder unverzweigt), oder CO(OR') (mit R' = C₁-C₈-Alkyl (verzweigt oder unverzweigt) steht, bedeutet,
R² Wasserstoff, COR⁶, wobei R⁶ für C₁-C₈-Alkyl(verzweigt oder unverzweigt) steht, bedeutet,
R³ C₁-C₈-Alkyl (verzweigt oder unverzweigt), C₃-C₈-Cycloalkyl, gegebenenfalls substituiertes Phenyl, gegebenenfalls substituiertes 1-Naphtyl, 2-Naphtyl, Chinolin, Anthracen, Phenanthren, Benzothiophen, Benzofurfuryl, gegebenenfalls substituiertes Pyrrol, 2-Pyridyl, 3-Pyridyl, 4-Pyridyl, gegebenenfalls substituiertes Furfuryl oder gegebenenfalls substituiertes Thiophen bedeutet,
X CR⁵, N oder S bedeutet und Y für den Fall, daß X S bedeutet, CR⁶ oder N und in allen anderen Fällen N bedeutet, wobei die strichelung im Strukturelement 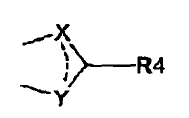 bedeutet, daß in den Fällen, wo X S bedeutet, Y über eine Doppelbindung mit dem R⁴ tragenden C-Atom verknüpft ist und in allen anderen Fällen eine der Gruppen X über eine Doppelbindung mit dem R⁴ tragenden C-Atom verknüpft ist und die jeweils andere einen zusätzlichen Wasserstoff trägt,
R⁴, R⁵ und R⁶ unabhängig voneinander Wasserstoff, C₁-C₉-Alkyl (verzweigt oder unverzweigt), oder CH₂CO(OR'), wobei R' jeweils die oben angegebene Bedeutung hat oder auch Wasserstoff bedeutet und deren pharmazeutisch akzeptable Salze
   ausgenommen Verbindungen, bei denen entweder gleichzeitig R¹ C(CH₃)₃, R² Wasserstoff, R³ unsubstituiertes Phenyl, X S und Y N oder CR⁶ mit R⁶= Wasserstoff oder CH₂-CO₂-Ethyl oder gleichzeitig R¹ C(CH₃)₃, R² Wasserstoff, R³ unsubstituiertes Phenyl, Y NH und X N oder CR⁵ mit R⁵ = CO₂Ethyl bedeutet.

Für den Fall, daß R³ eine substituierte Phenylgruppe ist, ist diese ausgewählt aus der Gruppe 4-Acetamidophenyl, 2-Bromphenyl, 3-Bromphenyl, 4-Bromphenyl, 4-Brom-2-fluorphenyl, 5-Brom-2-fluorphenyl, 3-Brom-4-fluorphenyl, 4-*tert*. Butylphenyl, 2-Chlor-4-fluorphenyl, 2-Chlor-6-fluorphenyl, 2-Chlorphenyl, 3-Chlorphenyl, 4-Chlorphenyl, 4-Cyanophenyl, 2,3-Dichlorphenyl, 2,4-Dichlorphenyl, 3,4-Dichlorphenyl, 2,3-Dimethoxyphenyl, 3, 4-Dimethoxyphenyl, 2,4-Dimethylphenyl, 2,5-Dimethylphenyl, 2-Fluorphenyl, 3-Fluorphenyl, 4-Fluorphenyl, 4-Hexylphenyl, 3-Hydroxyphenyl, 2-Methoxyphenyl, 2-Methylphenyl, 3-Methylphenyl, 4-Methylphenyl, 4-Nitrophenyl, 3-Phenoxyphenyl, 4-(1-Pyrrolidino)phenyl, 2-(Trifluormethyl)phenyl, 3-(Tri-fluormethyl)phenyl, 4-(Trifluormethyl)phenyl, 3,4,5-Trimethoxyphenyl, 3- (4-Chlorphenoxy)phenyl, 4-Acetoxy-3-methoxyphenyl.

Für den Fall, daß R³ eine substituierte 1-Naphthylgruppe ist, ist diese ausgewählt aus der Gruppe 4-Dimethylaminonaphthyl, 2-Ethoxynaphthyl, 4-Methoxynaphthyl.

Für den Fall, daß R³ eine substituierte Pyrrolgruppe ist, ist diese ausgewählt aus der Gruppe 2-(1-(Phenylsulfonyl)-pyrrol), 2-(N-Methylpyrrol), 2-(N-(3,5-Dichlorphenyl)-pyrrol), 2(1-(4-Chlorphenyl)pyrrol).

Für den Fall, daß R³ eine substituierte Furfurylgruppe ist, ist diese ausgewählt aus der Gruppe 2-(5-Acetoxymethylfurfuryl), 2-(5-Methylfurfuryl), 2-(5-Nitrofurfuryl), 2-[5-(3-Nitrophenyl)furfuryl], 2-[5-(2-Nitro-phenyl)furfuryl], 2-(5-Bromfurfuryl), 2-[5-(4-Chlorphenyl)furfuryl], 2-(4,5-Dimethylfurfuryl), 2-[5-(2-Chlorphenyl]furfuryl], 2-(5-Ethylfurfuryl), 2-[5-(1,3-Dioxalan)furfuryl].

Für den Fall, daß R³ eine substituierte Thiophengruppe ist, diese ausgewählt ist aus der Gruppe 2-(5-Chlorthiophenyl), 2-(5-Methylthiophenyl), 2-(5-Ethylthiophenyl), 2-(3-Methylthiophenyl), 2-(4-Brom-thiophenyl), 2-(5-Nitrothiophenyl), 5-(2-Carbonsäurethiophenyl), 2-[4-(Phenylethyl)thiophenyl], 2-[5-(Methylthio)thiophenyl], 2-(3-Bromthiophenyl), 2-(3-Phenoxythiophenyl), 2-(5-Bromthiophenyl).

Besonders bevorzugt sind erfingdungsgemäß bicyclische Imidazo-5-yl-amine ausgewählt aus der Gruppe *tert*-Butyl-(5-furan-2-yl-imidazo[1,2-b][1,2,4]triazol-6-yl)-amin,
*tert*-Butyl-(6-furan-2-yl-imidazo[2,1-b]thiazol-5-yl)-amin,
(5-*tert*-Butylamino-6-furan-2-yl-imidazo[2,1-b]thiazol-3-yl)-essigsäure,
*tert*-Butyl-(5-pyridin-2-yl-imidazo[1,2-b] [1,2,4]triazol-6-yl)-amin,
*tert*-Butyl-(6-pyridin-2-yl-imidazo[2,1-b]thiazol-5-yl)-amin,
*tert*-Butyl-(5-pyridin-3-yl-imidazo[1,2-b] [1,2,4]triazol-6-yl)-amin,
*tert*-Butyl-(5-pyridin-4-yl-imidazo[1,2-b] [1,2,4]triazol-6-yl)-amin,
*tert*-Butyl-(6-cyclohexyl-imidazo[2,1-b]thiazol-5-yl)-amin,
*tert*-Butyl-(5-methyl-imidazo[1,2-b][1,2,4]triazol-6-yl)-amin,
*tert*-Butyl-(6-methyl-imidazo[2,1-b]thiazol-5-yl)-amin, Cyclohexyl-(5-pyridin-2-yl-imidazo[1,2-b]
[1,2,4]triazol-6-yl)-amin, Cyclohexyl-(6-pyridin-2-yl-imidazo[2,1-b]thiazol-5-yl)-amin,
(5-Cyclohexylamino-6-pyridin-2-yl-imidazo[2,1-b]thiazol-3-yl)-essigsäure,
Cyclohexyl-(6-pyridin-4-yl-imidazo[2,1-b]thiazol-5-yl)-amin,
Cyclohexyl-(6-cyclohexyl-imidazo[2,1-b]thiazol-5-yl)-amin,
(6-Cyclohexyl-5-cyclohexylamino-imidazo[2,1-b]thiazol-3-yl)-essigsäure,
(5-Cyclohexylamino-6-methyl-imidazo[2,1-b]thiazol-3-yl)-essig-säure,
(6-Cyclohexyl-imidazo[2,1-b]thiazol-5-ylamino)-essigsäuremethylester,
(6-Methyl-imidazo[2,1-b]thiazol-5-ylamino)-essigsäuremethylester,
*tert*-Butyl-(2-phenyl-5H-imidazo[1,2-b]pyrazol-3-yl)-amin,
3-(5-*tert*-Butylamino-imidazo[2,1-b]thiazol-6-yl)-phenol,
*tert*-Butyl-[6-(3,4-dimethoxy-phenyl)-imidazo[2,1-b]thiazol-5-yl]-amin,
*tert*-Butyl-[5-(2,3-dichlor-phenyl) -imidazo[1,2-b] [1,2,4]triazol-6-yl]-amin,
*tert*-Butyl-[6-(2,3-dichlor-phenyl)-imidazo[2,1-b]thiazol-5-yl]-amin,
*tert*-Butyl-[5-(2,4-dichlor-phenyl)-imidazo[1,2-b] [1,2,4]triazol-6-yl]-amin,
*tert*-Butyl-[6-(2,4-dichlor-phenyl)-imidazo[2,1-b]thiazol-5-yl]-amin,
*tert*-Butyl-[5-(2-methoxy-phenyl) -imidazo[1,2-b] [1,2,4]triazol-6-yl]-amin,
*tert*-Butyl-[6-(2-methoxy-phenyl)-imidazo[2,1-b]thiazol-5-yl]-amin,
[5-*tert*-Butylamino-6-(2-methoxy-phenyl)-imidazo[2,1-b] thiazol-3-yl]-essigsäure,
*tert*-Butyl-(5-o-tolyl-imidazo[1,2-b][1,2,4]triazol-6-yl)-amin,
*tert*-Butyl-(6-o-tolyl-imidazo[2,1-b]thiazol-5-yl)-amin,
*tert*-Butyl-[5-(2,3-dimethoxy-phenyl) -imidazo[1,2-b][1,2,4]triazol-6-yl]-amin,
*tert*-Butyl-[6-(2,3-dimethoxy-phenyl)-imidazo[2,1-b] thiazol-5-yl]-amin,
*tert*-Butyl-(6-p-tolyl-imidazo[2,1-b]thiazol-5-yl)-amin.
(5-*tert*-Butylamino-6-methyl-imidazo[2,1-b]thiazol-3-yl)-essigsäure,
N-*tert*-Butyl-N-(6-phenyl-imidazo[2,1-b]thiazol-5-yl)-acetamid,
N-*tert*-Butyl-N-(6-o-tolyl-imidazo[2,1-b]thiazol-5-yl)-acetamid,
Butyl-[6-(4-*tert*-butyl-phenyl)-2-methyl-imidazo[2,1-b]thiazol-5-yl]-amin,
*tert*-Butyl-[5-(2-fluorphenyl)-imidazo[1,2-b][1,2,4]triazol-6-yl]-amin,
*tert*-Butyl-[6-(2-fluorphenyl)-imidazo[2,1-b]thiazol-5-yl]-amin,
*tert*-Butyl-(5-naphthalen-1-yl-imidazo[1,2-b][1,2,4]triazol-6-yl)-amin,
Cyclohexyl-(5-naphthalin-1-yl-imidazo[1,2-b][1,2,4]triazol-6-yl)-amin,
[5-(2-Bromphenyl)-imidazo[1,2-b][1,2,4]triazol-6-yl]-(1,1,3,3-tetramethyl-butyl)-amin,
N-[4-(6-Cyclohexylamino-imidazo[1,2-b][1,2,4]triazol-5-yl)-phenyl]-acetamid,
*tert*-Butyl-[5-(2,5-dimethyl-phenyl)-imidazo[1,2-b][1,2,4]triazol-6-yl]-amin,
Cyclohexyl-[6-(2,4-dimethyl-phenyl)-imidazo[2,1-b]thiazol-5-yl]-amin,
Cyclohexyl-[6-(2,5-dimethylphenyl)-imidazo[2,1-b]thiazol-5-yl]-amin,
N-*tert*-Butyl-*N*-(6-p-tolyl-imidazo[2,1-b]thiazol-5-yl)-acetamid,
[5-(2,4-Dimethyl-phenyl) -imidazo[1,2-b][1,2,4]triazol-6-yl]-(1,1,3,3-tetramethyl-butyl)-amin,
[5-(2,5-Dimethyl-phenyl)-imidazo[1,2-b][1,2,4]triazol-6-yl]-(1,1,3,3-tetramethyl-butyl)-amin,
N-Butyl-N-[5-(2-chlor-6-fluorphenyl) -imidazo[1,2-b][1,2,4]triazol-6-yl]-acetamid und
N-Butyl-N-[6-(4-tert-butyl-phenyl)-2-methylimidazo[2,1-b]thiazol-5-yl]-acetamid.

Soweit die erfindungsgemäßen bicyclischen Imidazo-5-yl-amine optisch aktive Kohlenstoffatome enthalten, sind auch die Enantiomeren dieser Verbindungen und deren Mischungen sowie deren pharmazeutisch akzeptable Salze Gegenstand der vorliegenden Erfindung.

Gegenstand der Erfindung sind außerdem Arzneimittel enthaltend als Wirkstoff mindestens ein bicyclisches Imidazo-5-yl-amin der allgemeinen Formel I, in der R¹ bis R⁶, X und Y die oben angegebene Bedeutung haben,in Form der Base oder von pharmazeutisch akzeptablen Salzen, vorzugsweise der Bromwasserstoffsäure, Schwefelsäure, Methansulfonsäure, Ameisensäure, Essigsäure, Oxalsäure, Bernsteinsäure, Weinsäure, Mandelsäure, Furmarsäure, Milchsäure, Zitronensäure, Glutaminsäure und/ oder Asparaginsäure oder insbesondere der Salzsäure.

Besonders bevorzugt enthalten die erfindungsgemäßenen Arzneimittel als Wirkstoff mindestens ein bicyclisches Imidazo-5-yl-amin ausgewählt aus der Gruppe
*tert*-Butyl-(5-furan-2-yl-imidazo[1,2-b][1,2,4]triazol-6-yl)-amin,
*tert*-Butyl-(6-furan-2-yl-imidazo[2,1-b]thiazol-5-yl)-amin,
(5-*tert*-Butylamino-6-furan-2-yl-imidazo[2,1-b]thiazol-3-yl)-essigsäure,
*tert*-Butyl-(5-pyridin-2-yl-imidazo[1,2-b] [1,2,4]triazol-6-yl)-amin,
*tert*-Butyl-(6-pyridin-2-yl-imidazo[2,1-b]thiazol-5-yl)-amin,
*tert*-Butyl-(5-pyridin-3-yl-imidazo[1,2-b] [1,2,4]triazol-6-yl)-amin,
*tert*-Butyl-(5-pyridin-4-yl-imidazo[1,2-b] [1,2,4]triazol-6-yl)-amin,
*tert*-Butyl-(6-cyclohexyl-imidazo[2,1-b]thiazol-5-yl)-amin,
*tert*-Butyl-(5-methyl-imidazo[1,2-b][1,2,4]triazol-6-yl)-amin,
*tert*-Butyl-(6-methyl-imidazo[2,1-b]thiazol-5-yl)-amin,
Cyclohexyl-(5-pyridin-2-yl-imidazo[1,2-b] [1,2,4]triazol-6-yl)-amin,
Cyclohexyl-(6-pyridin-2-yl-imidazo[2,1-b]thiazol-5-yl)-amin,
(5-Cyclohexylamino-6-pyridin-2-yl-imidazo[2,1-b]thiazol-3-yl)-essigsäure,
Cyclohexyl-(6-pyridin-4-yl-imidazo[2,1-b]thiazol-5-yl)-amin,
Cyclohexyl-(6-cyclohexyl-imidazo[2,1-b]thiazol-5-yl)-amin,
(6-Cyclohexyl-5-cyclohexylamino-imidazo[2,1-b]thiazol-3-yl)-essigsäure,
(5-Cyclohexylamino-6-methyl-imidazo[2,1-b]thiazol-3-yl)-essig-säure,
(6-Cyclohexyl-imidazo[2,1-b]thiazol-5-ylamino)-essigsäuremethylester,
(6-Methyl-imidazo[2,1-b]thiazol-5-ylamino)-essigsäuremethylester,
tert-Butyl-(2-phenyl-5H-imidazo[1,2-b]pyrazol-3-yl)-amin,
3-(5-tert-Butylamino-imidazo[2,1-b]thiazol-6-yl)-phenol,
tert-Butyl-[6-(3,4-dimethoxy-phenyl)-imidazo[2,1-b]thiazol-5-yl]-amin,
tert-BuLyl-[5-(2,3-dichlor-phenyl) -imidazo[1,2-b] [1,2,4]triazol-6-yl]-amin,
tert-Butyl-[6-(2,3-dichlor-phenyl)-imidazo[2,1-b]thiazol-5-yl]-amin,
tert-Butyl-[5-(2,4-dichlor-phenyl) -imidazo[1,2-b] [1,2,4]triazol-6-yl]-amin,
*tert*-Butyl-[6-(2,4-dichlor-phenyl)-imidazo [2, 1-b]thiazol-5-yl]-amin,
*tert*-Butyl-[5-(2-methoxy-phenyl) -imidazo[1,2-b] [1,2,4]triazol-6-yl]-amin,
*tert*-Butyl-[6-(2-methoxy-phenyl)-imidazo[2,1-b]thiazol-5-yl]-amin,
[5-*tert*-Butylamino-6-(2-methoxy-phenyl)-imidazo[2,1-b] thiazol-3-yl]-essigsäure,
*tert*-Butyl-(5-o-tolyl-imidazo[1,2-b][1,2,4]triazol-6-yl)-amin,
*tert*-Butyl-(6-o-tolyl-imidazo[2,1-b]thiazol-5-yl)-amin,
*tert*-Butyl-[5-(2,3-dimethoxy-phenyl) -imidazo[1,2-b][1,2,4]triazol-6-yl]-amin,
*tert*-Butyl-[6-(2,3-dimethoxy-phenyl)-imidazo[2,1-b] thiazol-5-yl]-amin,
*tert*-Butyl-(6-p-tolyl-imidazo[2,1-b]thiazol-5-yl)-amin,
(5-*tert*-Butylamino-6-methyl-imidazo[2,1-b]thiazol-3-yl)-essigsäure,
N-*tert*-Butyl-N-(6-phenyl-imidazo[2,1-b]thiazol-5-yl)-acetamid,
N-*tert*-Butyl-N-(6-o-tolyl-imidazo[2,1-b]thiazol-5-yl)-acetamid,
Butyl-[6-(4-*tert*-butyl-phenyl)-2-methyl-imidazo[2,1-b]thiazol-5-yl]-amin,
*tert*-Butyl-[5-(2-fluorphenyl)-imidazo[1,2-b][1,2,4]triazol-6-yl]-amin,
*tert*-Butyl-[6-(2-fluorphenyl)-imidazo[2,1-b]thiazol-5-yl]-amin,
*tert*-Buty1-(5-naphthalen-1-yl-imidazo[1,2-b][1,2,4]triazol-6-yl)-amin,
Cyclohexyl-(5-naphthalin-1-yl-imidazo[1,2-b][1,2,4]triazol-6-yl)-amin,
[5-(2-Bromphenyl)-imidazo[1,2-b][1,2,4]triazol-6-yl]-(1,1,3,3-tetramethyl-butyl)-amin,
N-[4-(6-Cyclohexylamino-imidazo[1,2-b][1,2,4]triazol-5-yl)-phenyl]-acetamid,
*tert*-Butyl-[5-(2,5-dimethyl-phenyl)-imidazo[1,2-b][1,2,4]triazol-6-yl]-amin,
Cyclohexyl-[6-(2,4-dimethyl-phenyl)-imidazo[2,1-b]thiazol-5-yl]-amin,
Cyclohexyl-[G-(2,5-dimethylphenyl)-imidazo[2,1-b]thiazol-5-yl]-amin,
N-*tert*-Butyl-N-(6-p-tolyl-imidazo[2,1-b]thiazol-5-yl)-acetamid,
[5-(2,4-Dimethyl-phenyl) -imidazo[1,2-b][1,2,4]triazol-6-yl]-(1,1,3,3-tetramethyl-butyl)-amin,
[5-(2,5-Dimethyl-phenyl)-imidazo[1,2-b][1,2,4]triazol-6-yl]-(1,1,3,3-tetramethyl-butyl)-amin,
N-Butyl-N-[5-(2-chlor-6-fluorphenyl) -imidazo[1,2-b][1,2,4]triazol-6-yl]-acetamid und
N-Butyl-N-[6-(4-tert-butyl-phenyl)-2-methylimidazo[2,1-b]thiazol-5-yl]-acetamid
in Form der Base oder von pharmazeutisch akzeptablen Salzen

Die erfindungsgemäßen Verbindungen erwiesen sich als Liganden des schmerzrelevanten α2-Subtyps des humanen α-adrenergen Rezeptors.Besonders bevorzugt ist daher die Verwendung der erfindungsgemäßen bicyclischen Imidazo-5-yl-amine zusammen mit einem oder mehreren Hilfsstoffen zur Herstellung eines Arzneimittels zur Bekämpfung von Schmerz.

Zur Herstellung entsprechender Arzneimittel werden neben mindestens einem erfindungsgemäßen Wirkstoff Trägermaterialien, Füllstoffe, Lösungsmittel, Verdünnungsmittel, Farbstoffe und/oder Bindemittel eingesetzt. Die Auswahl der Hilfsstoffe sowie die einzusetzenden Mengen derselben hängt davon ab, ob das Arzneimittel oral, intravenös, intraperitoneal, intradermal, intramuskulär, intranasal, buccal oder örtlich appliziert werden soll. Für orale Applikation eignen sich Zubereitungen in Form von Tabletten, Dragees, Kapseln, Granulaten, Tropfen, Säften und Sirupen, für die parenterale, topische und inhalative Applikation Lösungen, Suspensionen, leicht rekonstituierbare Trockenzubereitungen sowie Sprays. Erfindungsgemäße Wirkstoffe in einem Depot, in gelöster Form oder in einem Pflaster, gegebenenfalls unter Zusatz von die Hautpenetration fördernden Mittel, sind geeignete perkutane Applikationszubereitungen. Oral oder perkutan anwendbare Zubereitungsformen können die erfindungsgemäßen Wirkstoffe verzögert freisetzen.

Die an den Patienten zu verabreichende Wirkstoffmenge variiert in Abhängigkeit vom Gewicht des Patienten, von der Applikationsart, der Indikation und dem Schweregrad der Erkrankung.

Die Synthese der erfindungsgemäßen Verbindungen erfolgt in der Weise, daß man Amidine mit der allgemeinen Formel II, insbesondere 3-Aminopyrazol-, 3-Amino-1,2,4-triazol-, 2-Amino-, 1,3,4-Thiadiazol- und 2-Aminothiazolderivate, die von Firmen wie beispielsweise Acros, Avocado, Aldrich, Fluka, Lancaster, Maybridge, Merck, Sigma oder TCI-Jp kommerziell angeboten werden, mit verschiedensten Aldehyden III und Isonitrilen IV in Gegenwart von 20%-iger Perchlorsäure gemäß einer Dreikomponentenreaktion umsetzt. R1 bis R3, X und Y haben dabei die oben für Verbindungen der Formel I angegebene Bedeutung.

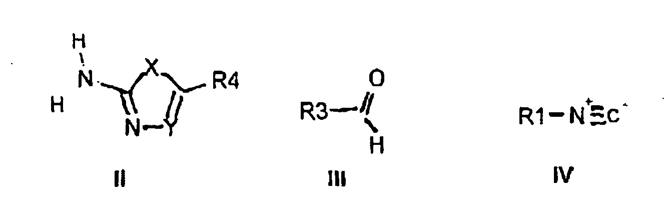

Vorzugsweise werden die Reaktionen in Dichlormethan (DCM) bei einer Temperatur von 0°c bis 40°C, insbesondere bei 10°C bis 20°C durchgeführt.

Zur Herstellung der erfindungsgemäßen Verbindungen, in denen R² nicht Wasserstoff bedeutet, werden die in der zuvor beschriebenen Reaktion entstehenden Verbindungen Ia, die vorzugsweise zunächst in Dichlormethan oder THF gelöst wurden, je nach gewünschtem Endprodukt mit einer Verbindung R²Hal, wobei Hal für Brom, Iod oder insbesondere Chlor steht, beispielsweise einem gegebenenfalls substituierten Alkyl-, Aryl- oder Säurechlorid, in Gegenwart eines Morpholin-Harzes (z.B. Polystyrol-Morpholin der Firma Argonaut) in Dichlormethan innerhalb von 2 bis 24 Stunden bei Temperaturen zwischen 10°C und 40°C gemäß dem folgenden Reaktionsschema umgesetzt:

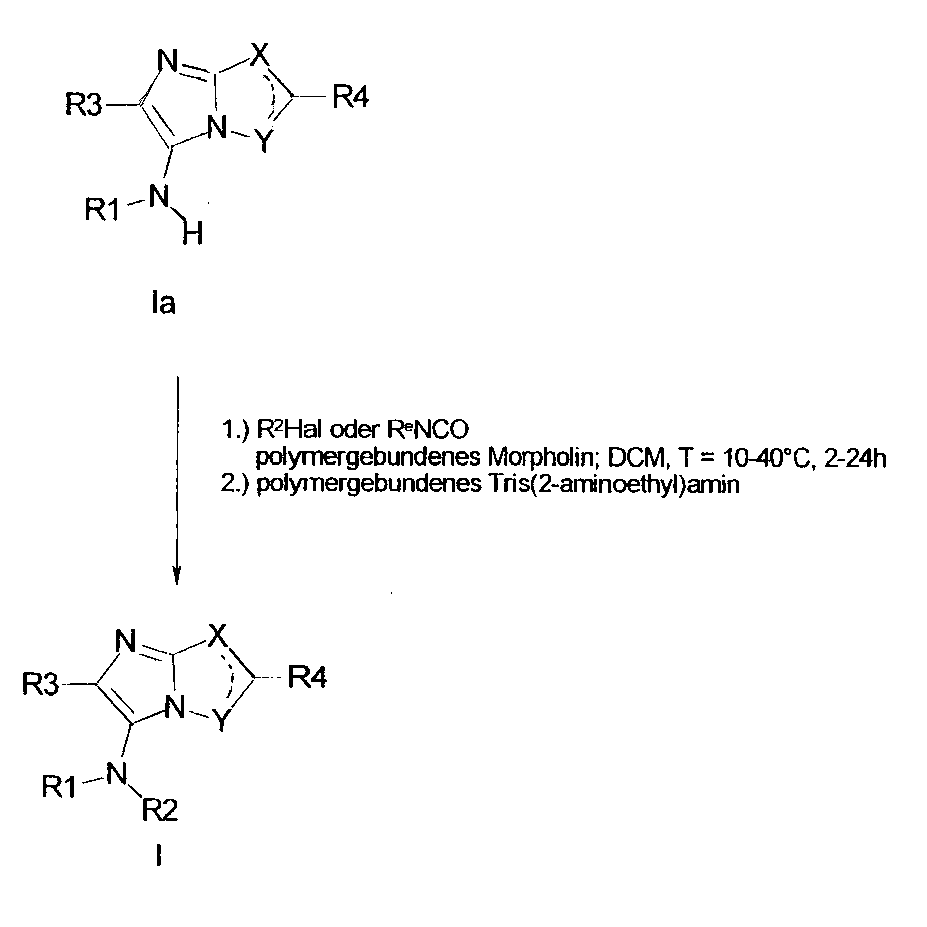

Die überschüssigen Reagentien werden anschließend durch Filtration über eine Schicht mit polymergebundenem Tris(2-aminoethyl)amin (Hersteller: Novabiochem) oder 3-(3-Mercaptophenyl)propanamidomethylpolystyrol aus dem Reaktionsgemisch entfernt und das Filtrat vorzugsweise in einer Vakuumzentrifuge aufkonzentriert. Das gesamte Verfahren läßt sich ohne weiteres auch in einer automatisierten Syntheseanlage durchführen. Die Verbindungen der Formel I lassen sich mit physiologisch verträglichen Säuren, vorzugsweise Bromwasserstoffsäure, Schwefelsäure, Methansulfonsäure, Ameisensäure, Essigsäure, Oxalsäure, Bernsteinsäure, Weinsäure, Mandelsäure, Furmarsäure, Milchsäure, Zitronensäure, Glutaminsäure und/ oder Asparaginsäure und insbesondere Salzsäure, in der an sich bekannten Weise in ihre pharmazeutisch akzeptablen Salze überführen. Vorzugsweise wird die Salzbildung in einem Lösungsmittel, insbesondere Diethylether, Diisopropylether, Essigsäurealkylester, Aceton oder 2-Butanon oder einem Gemisch dieser Lösungsmittel durchgeführt. Zur Herstellung der Hydrochloride eignet sich alternativ auch Trimethylsilan in wässriger Lösung.

### Beispiele:

Die folgenden Beispiele sollen die Erfindung erläutern, ohne sie darauf zu beschränken.

Die Synthese der erfolgte auf einer automatischen Anlage der Firma Zymark nach folgender allgemeiner Synthesevorschrift:

Ein Rundbodenröhrchen aus Glas (Durchmesser 16 mm, Länge 125 mm) mit Gewinde wurde manuell mit einem Rührer versehen und auf der Capper-Station mit einem Schraubdeckel mit Septum verschlossen. Das Röhrchen wurde von Roboter 1 in den auf 15°C temperierten Reaktorblock gestellt. Roboter 2 pipettierte nacheinander folgende Reagenzien hinzu:
1.) 1 ml einer 0,1 M Amidin-Lösung + 20% HClO₄ in Dichlormethan
2.) 0,5 ml einer 0,3 M Aldehyd-Lösung in Dichlormethan
3.) 0,575 ml einer 0,2 M Isonitril-Lösung in Dichlormethan

Das Reaktionsgemisch wurde bei 15°C in einem der Rührblöcke 660 min lang gerührt. Danach wurde die Reaktionslösung an der Filtrations-Station abfiltriert. Das Röhrchen wurde dabei zweimal mit je 1 ml Dichlormethan und 200 µl Wasser gespült.

Das Rack mit den Röhrchen wurde anschließend manuell auf die Aufarbeitungsanlage gestellt. Dort wurde das Reaktionsgemisch auf einem Vortexer mit 3 ml einer 10%igen NaCl-Lösung und 1,5 ml Dichlormethan versetzt. Im Spin-Reaktor wurde zehn Minuten lang gründlich gemischt und durch die langsame Abnahme der Drehbewegung eine deutliche Phasengrenze ausgebildet. Diese Phasengrenze wurde optisch detektiert und die organische Phase abpipettiert. Im nächsten Schritt wurde das Reaktionsgemisch erneut mit 1,5 ml Dichlormethan versetzt. Die Lösung wurde geschüttelt, zentrifugiert und die organische Phase abpipettiert. Die vereinigten organischen Phasen wurden über 2,4 g MgSO₄ (granuliert) getrocknet. Das Lösungsmittel wurde in einer Vakuumzentrifuge entfernt.

Für die Beispiele, in denen die so entstandene Verbindung weiter mit Acetylchlorid umgesetzt wurde, geschah dies nach folgender allgemeinen Vorschrift:

Das nach der vorstehenden allgemeinen Synthesevorschrift erhaltene Produkt wurde in Dichlormethan gelöst, mit 4 Moläquivalenten

Acetylchlorid versetzt und vier Stunden bei 18 °C gerührt. Das überschüssige Acetylchlorid und das Lösungsmittel wurden bei 40-60°C im Vakuum entfernt.

Die eingesetzten Chemikalien und Lösungsmittel wurden kommerziell erworben. Jede Substanz wurde mit ESI-MS und/oder NMR analysiert.

### Beispiel 1

### tert-Butyl-(5-furan-2-yl-imidazo[1,2-b][1,2,4]triazol-6-yl)-amin (1)

Verbindung **1** wurde gemäß der allgemeinen Synthesevorschrift aus 1.0 ml (0.1 mmol) 3-Amino-1,2,4-triazol-Lösung (0.1M, DCM), 0.575 ml (0.115 mmol) *tert.*-Butylisonitril-Lösung (0.2M, DCM), 0.500 ml (0.15 mmol) Furfural-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 262

### Beispiel 2

### tert-Butyl-(6-furan-2-yl-imidazo[2,1-b]thiazol-5-yl)-amin (2)

Verbindung **2** wurde gemäß der allgemeinen Synthesevorschrift aus 1.0 ml (0.1 mmol) 2-Aminothiazol-Lösung (0.1M, DCM), 0.575 ml (0.115 mmol) *tert.*-Butylisonitril-Lösung (0.2M, DCM), 0.500 ml (0.15 mmol) Furfural-Lösung (0.3 M, DCM) und 10 µl Perchlorsäure (w = 20%) in einer Substanzbibliothek dargestellt.

Zur Charakterisierung wurde ein ESI-MS aufgenommen Gefundene Masse: 262

### Beispiel 3

### (5-tert-Butylamino-6-furan-2-yl-imidazo[2,1-b]thiazol-3-yl)-essigsäure (3)

Verbindung **3** wurde gemäß der allgemeinen Synthesevorschrift aus 1.0 ml (0.1 mmol) (2-Aminothiazol-4-yl)essigsäure-Lösung (0.1M, DCM), 0.575 ml (0.115 mmol) *tert.*-Butylisonitril-Lösung (0.2M, DCM), 0.500 ml (0.15 mmol) Furfural-Lösung (0.3 M, DCM) und 10 µl Perchlorsäure (w = 20%) in einer Substanzbibliothek dargestellt.
Zur Charakterisierung wurde ein CSI-MS aufgenommen Gefundene Masse: 320

### Beispiel 4

### tert-Butyl-(5-pyridin-2-yl-imidazo[1,2-b][1,2,4]triazol-6-yl)-amin (4)

Verbindung **4** wurde gemäß der allgemeinen Synthesevorschrift aus 1.0 ml (0.1 mmol) 3-Amino-1,2,4-triazol-Lösung (0.1M, DCM), 0.575 ml (0.115 mmol) *tert.*-Butylisonitril-Lösung (0.2M, DCM), 0.500 ml (0.15 mmol) 2-Pyridincarbaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 257

### Beispiel 5

### tert-Butyl-(6-pyridin-2-yl-imidazo[2,1-b]thiazol-5-yl)-amin (5)

Verbindung **5** wurde gemäß der allgemeinen Synthesevorschrift aus 1.0 ml (0.1 mmol) 2-Aminothiazol-Lösung (0.1M, DCM), 0.575 ml (0.115 mmol) tert. Butylisonitril-Lösung (0.2M, DCM), 0.500 ml (0.15 mmol) 2-Pyridincarbaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 273

### Beispiel 6

### tert-Butyl-(5-pyridin-3-yl-imidazo[1,2-b][1,2,4]triazol-6-yl)-amin (6)

Verbindung **6** wurde gemäß der allgemeinen Synthesevorschrift aus 1.0 ml (0.1 mmol) 3-Amino-1,2,4-triazol-Lösung (0.1M, DCM), 0.575 ml (0.115 mmol) *tert.*-Butylisonitril-Lösung (0.2M, DCM), 0.500 ml (0.15 mmol) 3-Pyridincarbaldehyd-Lösung (0.3M, DCM) und 10 µl Perchlorsäure (w = 20%) in einer Substanzbibliothek dargestellt.
Zur Charakterisierung wurde ein ESI-MS aufgenommen Gefundene Masse: 257

### Beispiel 7

### tert-Butyl-(5-pyridin-4-yl-imidazo[1,2-b][1,2,4]triazol-6-yl)-amin (7)

Verbindung **7** wurde gemäß der allgemeinen Synthesevorschrift aus 1.0 ml (0.1 mmol) 3-Amino-1,2,4-triazol-Lösung (0.1M, DCM), 0.575 ml (0.115 mmol) *tert.*-Butylisonitril-Lösung (0.2M, DCM), 0.500 ml (0.15 mmol) 4-Pyridincarbaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 257

### Beispiel 8

### tert-Butyl-(6-cyclohexyl-imidazo[2,1-b]thiazol-5-yl)-amin (8)

Verbindung **8** wurde gemäß der allgemeinen Synthesevorschrift aus 1.0 ml (0.1 mmol) 2-Aminothiazol-Lösung (0.1M, DCM), 0.575 ml (0.115 mmol) *tert.*-Butylisonitril-Lösung (0.2M, DCM), 0.500 ml (0.15 mmol) Cyclohexylcarbaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 278

### Beispiel 9

### tert-Butyl-(5-methyl-imidazo[1,2-b][1,2,4]triazol-6-yl)-amin (9)

Verbindung **9** wurde gemäß der allgemeinen Synthesevorschrift aus 1.0 ml (0.1 mmol) 3-Amino-1,2,4-triazol-Lösung (0.1M, DCM), 0.575 ml (0.115 mmol) *tert.*-Butylisonitril-Lösung (0.2M, DCM), 0.500 ml (0.15 mmol) Acetaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 194

### Beispiel 10

### tert-Butyl-(6-methyl-imidazo[2,1-b]thiazol-5-yl)-amin (10)

Verbindung **10** wurde gemäß der allgemeinen Synthesevorschrift aus 1.0 ml (0.1 mmol) 2-Aminothiazol-Lösung (0.1M, DCM), 0.575 ml (0.115 mmol) *tert.*-Butylisonitril-Lösung (0.2M, DCM), 0.500 ml (0.15 mmol) Aldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 210

### Beispiel 11

### Cyclohexyl-(5-pyridin-2-yl-imidazo[1,2-b][1,2,4]triazol-6-yl)-amin (11)

Verbindung **11** wurde gemäß der allgemeinen Synthesevorschrift aus 1.0 ml (0.1 mmol) 3-Amino-1,2,4-triazol-Lösung (0.1M, DCM), 0.575 ml (0.115 mmol) Cyclohexylisonitril-Lösung (0.2M, DCM), 0.500 ml (0.15 mmol) 2-Pyridincarbaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 283

### Beispiel 12

### Cyclohexyl-(6-pyridin-2-yl-imidazo[2,1-b]thiazol-5-yl)-amin (12)

Verbindung **12** wurde gemäß der allgemeinen Synthesevorschrift aus 1.0 ml (0.1 mmol) 2-Aminothiazol-Lösung (0.1M, DCM), 0.575 ml (0.115 mmol) Cyclohexylisonitril-Lösung (0.2M, DCM), 0.500 ml (0.15 mmol) 2-Pyridincarbaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 299

### Beispiel 13

### (5-Cyclohexylamino-6-pyridin-2-yl-imidazo[2,1-b]thiazol-3-yl)-essigsäure (13)

Verbindung **13** wurde gemäß der allgemeinen Synthesevorschrift aus 1.0 ml (0.1 mmol) (2-Aminothiazol-4-yl)essigsäure-Lösung (0.1M, DCM), 0.575 ml (0.115 mmol) Cyclohexylisonitril-Lösung (0.2M, DCM), 0.500 ml (0.15 mmol) 3-Pyridincarbaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 357

### Beispiel 14

### Cyclohexyl-(6-pyridin-4-yl-imidazo[2,1-b]thiazol-5-yl)-amin (14)

Verbindung **14** wurde gemäß der allgemeinen Synthesevorschrift aus 1.0 ml (0.1 mmol) 2-Aminothiazol-Lösung (0.1M, DCM), 0.575 ml (0.115 mmol) Cyclohexylisonitril-Lösung (0.2M, DCM), 0.500 ml (0.15 mmol) 3-Pyridincarbaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 299

### Beispiel 15

### Cyclohexyl-(6-cyclohexyl-imidazo[2,1-b]thiazol-5-yl)-amin (15)

Verbindung **15** wurde gemäß der allgemeinen Synthesevorschrift aus 1.0 ml (0.1 mmol) 2-Aminothiazol-Lösung (0.1M, DCM), 0.575 ml (0.115 mmol) Cyclohexylisonitril-Lösung (0.2M, DCM), 0.500 ml (0.15 mmol) Cyclohexylcarbaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 304

### Beispiel 16

### (6-Cyclohexyl-5-cyclohexylamino-imidazo[2,1-b]thiazol-3-yl)-essigsäure (16)

Verbindung **16** wurde gemäß der allgemeinen Synthesevorschrift aus 1.0 ml (0.1 mmol) (2-Aminothiazol-4-yl)essigsäure-Lösung (0.1M, DCM), 0.575 ml (0.115 mmol) Cyclohexylisonitril-Lösung (0.2M, DCM), 0.500 ml (0.15 mmol) Cyclohexylcarbaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 318

### Beispiel 17

### (5-Cyclohexylamino-6-methyl-imidazo[2,1-b]thiazol-3-yl)-essig-säure (17)

Verbindung **17** wurde gemäß der allgemeinen Synthesevorschrift aus 1.0 ml (0.1 mmol) (2-Aminothiazol-4-yl)essigsäure-Lösung (0.1M, DCM), 0.575 ml (0.115 mmol) Cyclohexylisonitril-Lösung (0.2M, DCM), 0.500 ml (0.15 mmol) Acetaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 250

### REFERENZBeispiel 18

### (2,6-Dimethyl-phenyl)-(5-furan-2-yl-imidazo[1,2-b] [1,2,4]triazol-6-yl)-amin (18)

Verbindung **18** wurde gemäß der allgemeinen Synthesevorschrift aus 1.0 ml (0.1 mmol) 3-Amino-1,2,4-triazol-Lösung (0.1M, DCM), 0.575 ml (0.115 mmol) 2,6-Dimethylphenylisonitril-Lösung (0.2M, DCM), 0.500 ml (0.15 mmol) Furfural-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 292

### REFERENZ- Beispiel 19

### (2,6-Dimethyl-phenyl)-(6-pyridin-2-yl-imidazo[2,1-b]thiazol-5-yl)-amin (19)

Verbindung **19** wurde gemäß der allgemeinen Synthesevorschrift aus 1.0 ml (0.1 mmol) 2-Aminothiazol-Lösung (0.1M, DCM), 0.575 ml (0.115 mmol) 2,6-Dimethylphenylisonitril-Lösung (0.2M, DCM), 0.500 ml (0.15 mmol) 2-Pyridincarbaldehyd-Lösung (0.3 M, DCM) und 10 µl Perchlorsäure (w = 20%) in einer substanzbibliothek dargestellt.
Zur Charakterisierung wurde ein ESI-MS aufgenommen Gefundene Masse: 321

### REFERENZBeispiel 20

### (2,6-Dimethyl-phenyl)-(6-pyridin-3-yl-imidazol2,1-b]thiazol-5-yl)-amin (20)

Verbindung **20** wurde gemäß der allgemeinen Synthesevorschrift aus 1.0 ml (0.1 mmol) 2-Aminothiazol-Lösung (0.1M, DCM), 0.575 ml (0.115 mmol) 2,6-Dimethylphenylisonitril-Lösung (0.2M, DCM), 0.500 ml (0.15 mmol) 3-Pyridincarbaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 321

### REFERENZBeispiel 21

### (2,6-Dimethyl-phenyl)-(6-pyridin-4-yl-imidazo[2,1-b]thiazol-5-yl)-amin (21)

Verbindung **21** wurde gemäß der allgemeinen Synthesevorschrift aus 1.0 ml (0.1 mmol) 2-Aminothiazol-Lösung (0.1M, DCM), 0.575 ml (0.115 mmol) 2,6-Dimethylphenylisonitril-Lösung (0.2M, DCM), 0.500 ml (0.15 mmol) 4-Pyridincarbaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 321

### Beispiel 22

### (6-Cyclohexyl-imidazo[2,1-b]thiazol-5-ylamino)-essigsäure- methylester (22)

Verbindung **22** wurde gemäß der allgemeinen Synthesevorschrift aus 1.0 ml (0.1 mmol) 2-Aminothiazol-Lösung (0.1M, DCM), 0.575 ml (0.115 mmol) Isocyanoessigsäuremethylester-Lösung (0.2M, DCM), 0.500 ml (0.15 mmol) Cyclohexylcarbaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 294

### Beispiel 23

### (6-Methyl-imidazo[2,1-b]thiazol-5-ylamino)-essigsäuremethylester (23)

Verbindung **23** wurde gemäß der allgemeinen Synthesevorschrift aus 1.0 ml (0.1 mmol) 2-Aminothiazol-Lösung (0.1M, DCM), 0.575 ml (0.115 mmol) Isocyanoessigsäuremethylester-Lösung (0.2M, DCM), 0.500 ml (0.15 mmol) Acetaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 226

### Beispiel 24

### tert-Butyl-(2-phenyl-5H-imidazo[1,2-b]pyrazol-3-yl)-amin(24)

Verbindung **24** wurde gemäß der allgemeinen Synthesevorschrift aus 1.0 ml (0.1 mmol) 3-Aminopyrazol-Lösung (0.1M, DCM), 0.575 ml (0.115 mmol) *tert.*-Butylisonitril-Lösung (0.2M, DCM), 0.500 ml (0.15 mmol) Benzaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 255

### Beispiel 25

### 3-(5-tert-Butylamino-imidazo[2,1-b]thiazol-6-yl)-phenol (25)

Verbindung **25** wurde gemäß der allgemeinen Synthesevorschrift aus 1.0 ml (0.1 mmol) 2-Aminothiazol-Lösung (0.1M, DCM), 0.575 ml (0.115 mmol) *tert.*-Butylisonitril-Lösung (0.2M, DCM), 0.500 ml (0.15 mmol) 3-Hydroxybenzaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 288

### Beispiel 26

### tert-Butyl-[6-(3,4-dimethoxy-phenyl)-imidazo[2,1-b]thiazol-5-yl]-amin (26)

Verbindung **26** wurde gemäß der allgemeinen Synthesevorschrift aus 1.0 ml (0.1 mmol) 2-Aminothiazol-Lösung (0.1M, DCM), 0.575 ml (0.115 mmol) *tert.*-Butylisonitril-Lösung (0.2M, DCM), 0.500 ml (0.15 mmol) 3,4-Dimethoxybenzaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 332

### Beispiel 27

### tert-Butyl-[5-(2,3-dichlor-phenyl) -imidazo[1,2-b] [1,2,4]triazol-6-yl]-amin(27)

Verbindung **27** wurde gemäß der allgemeinen Synthesevorschrift aus 1.0 ml (0.1 mmol) 3-Amino-1,2,4-triazol-Lösung (0.1M, DCM), 0.575 ml (0.115 mmol) *tert.*-Butylisonitril-Lösung (0.2M, DCM), 0.500 ml (0.15 mmol) 2,3-Dichlorbenzaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 324

### Beispiel 28

### tert-Butyl-[6-(2,3-dichlor-phenyl)-imidazo[2,1-b]thiazol-5-yl]-amin (28)

Verbindung **28** wurde gemäß der allgemeinen Synthesevorschrift aus 1.0 ml (0.1 mmol) 2-Aminothiazol-Lösung (0.1M, DCM), 0.575 ml (0.115 mmol) *tert.*-Butylisonitril-Lösung (0.2M, DCM), 0.500 ml (0.15 mmol) 2,3-Dichlorbenzaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 340

### Beispiel 29

### tert-Butyl-[5-(2,4-dichlor-phenyl) -imidazo[1,2-b] [1,2,4]triazol-6-yl]-amin (29)

Verbindung **29** wurde gemäß der allgemeinen Synthesevorschrift aus 1.0 ml (0.1 mmol) 3-Amino-1,2,4-triazol-Lösung (0.1M, DCM), 0.575 ml (0.115 mmol) *tert.*-Butylisonitril-Lösung (0.2M, DCM), 0.500 ml (0.15 mmol) 2,4-Dichlorbenzaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 324

### Beispiel 30

### tert-Butyl-[6-(2,4-dichlor-phenyl)-imidazo[2,1-b]thiazol-5-yl]-amin (30)

Verbindung **30** wurde gemäß der allgemeinen Synthesevorschrift aus 1.0 ml (0.1 mmol) 2-Aminothiazol-Lösung (0.1M, DCM), 0.575 ml (0.115 mmol) *tert.*-Butylisonitril-Lösung (0.2M, DCM), 0.500 ml (0.15 mmol) 2,4-Dichlorbenzaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 340

### Beispiel 31

### tert-Butyl-[5-(2-methoxy-phenyl) imidazo[1,2b][1,2,4]triazol-6-yl]-amin (31)

Verbindung **31** wurde gemäß der allgemeinen Synthesevorschrift aus 1.0 ml (0.1 mmol) 3-Amino-1,2,4-triazol-Lösung (0.1M, DCM), 0.575 ml (0.115 mmol) *tert.*-Butylisonitril-Lösung (0.2M, DCM), 0.500 ml (0.15 mmol) 2-Methoxybenzaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 286

### Beispiel 32

### tert-Butyl-[6-(2-methoxy-phenyl)-imidazo[2,1-b]thiazol-5-yl]-amin (32)

Verbindung **32** wurde gemäß der allgemeinen Synthesevorschrift aus 1.0 ml (0.1 mmol) 2-Aminothiazol-Lösung (0.1M, DCM), 0.575 ml (0.115 mmol) *tert.*-Butylisonitril-Lösung (0.2M, DCM), 0.500 ml (0.15 mmol) 2-Methoxybenzaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 302

### Beispiel 33

### [5-tert-Butylamino-6-(2-methoxy-phenyl)-imidazo[2,1-b]thiazol-3-yl]-essigsäure (33)

Verbindung **33** wurde gemäß der allgemeinen Synthesevorschrift aus 1.0 ml (0.1 mmol) (2-Aminothiazol-4-yl)essigsäure-Lösung (0.1M, DCM), 0.575 ml (0.115 mmol) *tert.*-Butylisonitril-Lösung (0.2M, DCM), 0.500 ml (0.15 mmol) 2-Methoxybenzaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 321

### Beispiel 34

### tert-Butyl-(5-o-tolyl-imidazo[1,2-b][1,2,4]triazol-6-yl)-amin (34)

Verbindung **34** wurde gemäß der allgemeinen Synthesevorschrift aus 1.0 ml (0.1 mmol) 3-Amino-1,2,4-triazol-Lösung (0.1M, DCM), 0.575 ml (0.115 mmol) *tert.*-Butylisonitril-Lösung (0.2M, DCM), 0.500 ml (0.15 mmol) 2-Methylbenzaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 270

### Beispiel 35

### tert-Butyl-(6-o-tolyl-imidazo[2,1-b]thiazol-5-yl)-amin (35)

Verbindung **35** wurde gemäß der allgemeinen Synthesevorschrift aus 1.0 ml (0.1 mmol) 2-Aminothiazol-Lösung (0.1.M, DCM), 0.575 ml (0.115 mmol) *tert.*-Butylisonitril-Lösung (0.2M, DCM), 0.500 ml (0.15 mmol) 2-Methylbenzaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 321

### Beispiel 36

### tert-Butyl-[5-(2,3-dimethoxy-phenyl) -imidazo[1,2-b] [1,2,4]triazol-6-yl]-amin (36)

Verbindung **36** wurde gemäß der allgemeinen Synthesevorschrift aus 1.0 ml (0.1 mmol) 3-Amino-1,2,4-triazol-Lösung (0.1M, DCM), 0.575 ml (0.115 mmol) *tert.*-Butylisonitril-Lösung (0.2M, DCM), 0.500 ml (0.15 mmol) 2,3-Dimethoxybenzaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 316

### Beispiel 37

### tert-Butyl-[6-(2,3-dimethoxy-phenyl)-imidazo[2,1-b]thiazol-5-yl]-amin (37)

Verbindung **37** wurde gemäß der allgemeinen Synthesevorschrift aus 1.0 ml (0.1 mmol) 2-Aminothiazol-Lösung (0.1M, DCM), 0.575 ml (0.115 mmol) *tert.*-Butylisonitril-Lösung (0.2M, DCM), 0.500 ml (0.15 mmol) 2,3-Dimethoxybenzaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 332

### Beispiel 38

### tert-Butyl-(6-p-tolyl-imidazo[2,1-b]thiazol-5-yl)-amin (38)

Verbindung **38** wurde gemäß der allgemeinen Synthesevorschrift aus 1.0 ml (0.1 mmol) 2-Aminothiazol-Lösung (0.1M, DCM), 0.575 ml (0.115 mmol) *tert.*-Butylisonitril-Lösung (0.2M, DCM), 0.500 ml (0.15 mmol) 4-Methylbenzaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 286

### Beispiel 39

### (5-tert-Butylamino-6-methyl-imidazo[2,1-b]thiazol-3-yl)-essigsäure (39)

Verbindung 39 wurde gemäß der allgemeinen Synthesevorschrift aus 1.0 ml (0.1 mmol) (2-Aminothiazol-4-yl)-essigsäure-Lösung (0.1M, DCM), 0.575 ml (0.115 mmol) *tert.*-Butylisonitril-Lösung (0.2M, DCM), 0.500 ml (0.15 mmol) Acetaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: M-CO₂ 224,3

### Beispiel 40:

### N-tert-Butyl-N-(6-phenyl-imidazo[2,1-b]thiazol-5-yl)-acetamid (40)

Verbindung 40 wurde gemäß der allgemeinen Synthesevorschrift aus 1.0 ml (0.1 mmol) 2-Aminothiazol-Lösung (0.1M, DCM), 0.575 ml (0.115 mmol) *tert.*-Butylisonitril-Lösung (0.2M, DCM), 0.500 ml (0.15 mmol) Benzaldehyd-Lösung (0.3 M, DCM) und 10 µl Perchlorsäure (w = 20%) und Umsetzung mit Acetylchlorid dargestellt, wobei das überschüssige Acetylchlorid im Vakuum entfernt wurde.
Zur Charakterisierung wurde ein ESI-MS aufgenommen Gefundene Masse: 315,3, M-Acetyl 272,1

### Beispiel 41

### N-tert-Butyl-N-(6-o-tolyl-imidazo[2,1-b]thiazol-5-yl)-acetamid (41)

Verbindung 41 wurde gemäß der allgemeinen Synthesevorschrift aus 1.0 ml (0.1 mmol) 2-Aminothiazol-Lösung (0.1M, DCM), 0.575 ml (0.115 mmol) *tert.*-Butylisonitril-Lösung (0.2M, DCM), 0.500 ml (0.15 mmol) 2-Methylbenzaldehyd-Lösung (0.3 M, DCM) und 10 µl Perchlorsäure (w = 20%) und Umsetzung mit Acetylchlorid dargestellt, wobei das überschüssige Acetylchlorid im Vakuum entfernt wurde.
Zur Charakterisierung wurde ein ESI-MS aufgenommen Gefundene Masse: M-Acetyl 286,3

### Beispiel 42

### Butyl-[6-(4-tert-butyl-phenyl)-2-methyl-imidazo[2,1-b]thiazol-5-yl]-amin (42)

Verbindung 42 wurde gemäß der allgemeinen Synthesevorschrift aus 1.0 ml (0.1 mmol) 5-Methylthiazol-2-yl-amin-Lösung (0.1M, DCM), 0.575 ml (0.115 mmol) n-Butylisonitril-Lösung (0.2M, DCM), 0.500 ml (0.15 mmol) 4-*tert*-Butylbenzaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 342,3

### Beispiel 43

### tert-Butyl-[5-(2-fluorphenyl)-imidazo[1,2-b][1,2,4]triazol-6-yl]-amin (43)

Verbindung 43 wurde gemäß der allgemeinen Synthesevorschrift aus 1.0 ml (0.1 mmol) 3-Amino-1,2,4-triazol-Lösung (0.1M, DCM), 0.575 ml (0.115 mmol) tert-Butylisonitril-Lösung (0.2M, DCM), 0.500 ml (0.15 mmol) 2-Fluorbenzaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 274,1

### Beispiel 44

### tert-Butyl-[6-(2-fluorphenyl)-imidazo[2,1-b]thiazol-5-yl]-amin (44)

Verbindung 44 wurde gemäß der allgemeinen Synthesevorschrift aus 1.0 ml (0.1 mmol) 2-Aminothiazol-Lösung (0.1M, DCM), 0.575 ml (0.115 mmol) *tert*-Butylisonitril-Lösung (0.2M, DCM), 0.500 ml (0.15 mmol) 2-Fluorbenzaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 290,2

### Beispiel 45

### tert-Butyl-(5-naphthalen-1-yl-imidazo[1,2-b][1,2,4]triazol-6-yl)-amin (45)

Verbindung 45 wurde gemäß der allgemeinen Synthesevorschrift aus 1.0 ml (0.1 mmol) 3-Amino-1,2,4-triazol-Lösung (0.1M, DCM), 0.575 ml (0.115 mmol) *tert-*Butylisonitril-Lösung (0.2M, DCM), 0.500 ml (0.15 mmol) 1-Naphtylcarbaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 306,2

### Beispiel 46

### Cyclohexyl-(5-naphthalin-1-yl-imidazo[1,2-b][1,2,4] triazol-6-yl) -amin (46)

Verbindung 46 wurde gemäß der allgemeinen Synthesevorschrift aus 1.0 ml (0.1 mmol) 3-Amino-1,2,4-triazol-Lösung (0.1M, DCM), 0.575 ml (0.115 mmol) Cyclohexylisonitril-Lösung (0.2M, DCM), 0.500 ml (0.15 mmol) 1-Naphtylcarbaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 332,3

### Beispiel 47

### [5-(2-Bromphenyl)-imidazo[1,2-b][1,2,4]triazol-6-yl]-(1,1,3,3-tetramethyl-butyl)-amin (47)

Verbindung 47 wurde gemäß der allgemeinen Synthesevorschrift aus 1.0 ml (0.1 mmol) 3-Amino-1,2,4-triazol-Lösung (0.1M, DCM), 0.575 ml (0.115 mmol) 1,1,3,3-Tetramethylbutylisonitril-Lösung (0.2M, DCM), 0.500 ml (0.15 mmol) 2-Brombenzaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 390,3/392,2

### Beispiel 48

### N-[4-(6-Cyclohexylamino-imidazo[1,2-b][1,2,4]triazol-5-yl)-phenyl]-acetamid (48)

Verbindung 48 wurde gemäß der allgemeinen Synthesevorschrift aus 1.0 ml (0.1 mmol) 3-Amino-1,2,4-triazol-Lösung (0.1M, DCM), 0.575 ml (0.115 mmol) Cyclohexylisonitril-Lösung (0.2M, DCM), 0.500 ml (0.15 mmol) N-(4-Formyl-phenyl)-acetamid-Lösung (0.3 M, DCM) und 10 µl Perchlorsäure (w = 20%) in einer Substanzbibliothek dargestellt.
Zur Charakterisierung wurde ein ESI-HS aufgenommen Gefundene Masse: 337,1

### Beispiel 49

### tert-Butyl-[5-(2,5-dimethyl-phenyl)-imidazo[1,2-b][1,2,4]triazol-6-yl]-amin (49)

Verbindung 49 wurde gemäß der allgemeinen Synthesevorschrift aus 1.0 ml (0.1 mmol) 3-Amino-1,2,4-triazol-Lösung (0.1M, DCM), 0.575 ml (0.115 mmol) *tert-*Butylisonitril-Lösung (0.2M, DCM), 0.500 ml (0.15 mmol) 2,5-Dimethylbenzaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 284,2

### Beispiel 50

### Cyclohexyl-[6-(2,4-dimethyl-phenyl)-imidazo[2,1-b]thiazol-5-yl]-amin (50)

Verbindung 50 wurde gemäß der allgemeinen Synthesevorschrift aus 1.0 ml (0.1 mmol) 2-Aminothiazol-Lösung (0.1M, DCM), 0.575 ml (0.115 mmol) Cyclohexylisonitril-Lösung (0.2M, DCM), 0.500 ml (0.15 mmol) 2,4-Dimethylbenzaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 326,3

### Beispiel 51

### Cyclohexyl-[6-(2,5-dimethylphenyl)-imidazo[2,1-b]thiazol-5-yl]-amin (51)

Verbindung 51 wurde gemäß der allgemeinen Synthesevorschrift aus 1.0 ml (0.1 mmol) 2-Aminothiazol-Lösung (0.1M, DCM), 0.575 ml (0.115 mmol) Cyclohexylisonitril-Lösung (0.2M, DCM), 0.500 ml (0.15 mmol) 2,5-Dimethylbenzaldehyd-Lösung (0.3 M, DCM) und 10 µl Perchlorsäure (w = 20%) in einer Substanzbibliothek dargestellt.
Zur Charakterisierung wurde ein ESI-MS aufgenommen Gefundene Masse: 326,3

### Beispiel 52

### N-tert-Butyl-N-(6-p-tolyl-imidazo[2,1-b]thiazol-5-yl)-acetamid (52)

Verbindung 52 wurde gemäß der allgemeinen Synthesevorschrift aus 1.0 ml (0.1 mmol) 2-Aminothiazol-Lösung (0.1M, DCM), 0.575 ml (0.115 mmol) *tert*-Butylisonitril-Lösung (0.2M, DCM), 0.500 ml (0.15 mmol) 4-Methylbenzaldehyd-Lösung (0.3 M, DCM) und 10 µl Perchlorsäure (w = 20%) und Umsetzung mit Acetylchlorid dargestellt, wobei das überschüssige Acetylchlorid im Vakuum entfernt wurde.
Zur Charakterisierung wurde ein ESI-MS aufgenommen Gefundene Masse: 327,4, M-Acetyl 286,3

### Beispiel 53

### [5-(2,4-Dimethyl-phenyl) -imidazo[1,2-b][1,2,4]triazol-6-yl]-(1,1,3,3-tetramethyl-butyl)-amin (53)

Verbindung 53 wurde gemäß der allgemeinen Synthesevorschrift aus 1.0 ml (0.1 mmol) 3-Amino-1,2,4-triazol -Lösung (0.1M, DCM), 0.575 ml (0.115 mmol) 1,1,3,3-Tetramethylbutylisonitril-Lösung (0.2M, DCM), 0.500 ml (0.15 mmol) 2,4-Dimethylbenzaldehyd-Lösung (0.3 M, DCM) und 10 µl Perchlorsäure (w = 20%) dargestellt.
Zur Charakterisierung wurde ein ESI-MS aufgenommen Gefundene Masse: 340,2

### Beispiel 54

### [5-(2,5-Dimethyl-phenyl)-imidazo[1,2-b][1,2,4]triazol-6-yl]-(1,1,3,3-tetramethyl-butyl)-amin (54)

Verbindung 54 wurde gemäß der allgemeinen Synthesevorschrift aus 1.0 ml (0.1 mmol) 3-Amino-1,2,4-triazol -Lösung (0.1M, DCM), 0.575 ml (0.115 mmol) 1,1,3,3-Tetramethylbutylisonitril-Lösung (0.2M, DCM), 0.500 ml (0.15 mmol) 2,5-Dimethylbenzaldehyd-Lösung (0.3 M, DCM) und 10 µl Perchlorsäure (w = 20%) dargestellt.
Zur Charakterisierung wurde ein ESI-MS aufgenommen Gefundene Masse: 340,2

### Beispiel 55

### N-Butyl-N-[5-(2-chlor-6-fluorphenyl) -imidazo[1,2-b][1,2,4]triazol-6-yl]-acetamid (55)

Verbindung 55 wurde gemäß der allgemeinen Synthesevorschrift aus 1.0 ml (0.1 mmol) 3-Amino-1,2,4-triazol -Lösung (0.1M, DCM), 0.575 ml (0.115 mmol) n-Butylisonitril-Lösung (0.2M, DCM), 0.500 ml (0.15 mmol) 2-Chlor-6-fluorbenzaldehyd-Lösung (0.3 M, DCM) und 10 µl Perchlorsäure (w = 20%) und Umsetzung mit Acetylchlorid dargestellt, wobei das überschüssige Acetylchlorid im Vakuum entfernt wurde.
Zur Charakterisierung wurde ein ESI-MS aufgenommen Gefundene Masse: 350,4

### Beispiel 56

### N-Butyl-N-[6-(4-tert-butyl-phenyl)-2-methylimidazo[2,1-b]thiazol-5-yl]-acetamid (56)

Verbindung 56 wurde gemäß der allgemeinen Synthesevorschrift aus 1.0 ml (0.1 mmol) 2-Amino-5-methylthiazol -Lösung (0.1M, DCM), 0.575 ml (0.115 mmol) n-Butylisonitril-Lösung (0.2M, DCM), 0.500 ml (0.15 mmol) 4-*tert*-Butylbenzaldehyd-Lösung (0.3 M, DCM) und 10 µl Perchlorsäure (w = 20%) und Umsetzung mit Acetylchlorid dargestellt, wobei das überschüssige Acetylchlorid im Vakuum entfernt wurde.
Zur Charakterisierung wurde ein ESI-MS aufgenommen Gefundene Masse: 384,5

Die erfindungsgemäßen Verbindungen erwiesen sich als Liganden des schmerzrelevanten α2-Subtyps des humanen α-adrenergen Rezeptors. Die Bestimmung der Affinität zum α2-Subtyps des humanen α-adrenergen Rezeptors erfolgte mittels eines für das High Throughput Screening üblichen SPA-Assays, wie er in John P. Devlin, High Throughput Screening, Marcel Dekker Inc. 1997, Seite 307 bis 316, beschrieben ist. Diese Literatur wird hiermit als Referenz eingeführt und gilt somit als Teil der Offenbarung. Bei einer Konzentration von 10 µM wurden die folgenden Affinitäten bestimmt:

| | alpha2-Affinität, 10µM |
|---|---|
| Beispiel 39 | 35% |
| Beispiel 40 | 77% |
| Beispiel 41 | 50% |
| Beispiel 42 | 36% |
| Beispiel 43 | 34% |
| Beispiel 44 | 38% |
| Beispiel 45 | 41% |
| Beispiel 46 | 46% |
| Beispiel 47 | 42% |
| Beispiel 48 | 36% |
| Beispiel 49 | 38% |
| Beispiel 50 | 36% |
| Beispiel 51 | 39% |
| Beispiel 52 | 51% |
| Beispiel 53 | 43% |
| Beispiel 54 | 56% |
| Beispiel 55 | 39% |
| Beispiel 56 | 46% |

## Patentansprüche

1. Bicyclische Imidazo-5-yl-amine der allgemeinen Formeln (I), 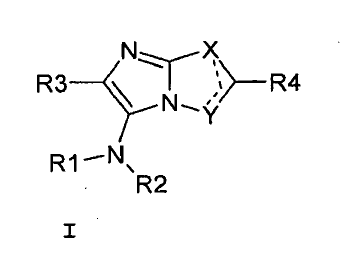 worin
R¹C(CH₃)₃, Phenyl, C₄-C₈-Cycloalkyl, 1,1,3,3-Tetramethylbutyl oder CH₂R^{a}, wobei R^{a} für Wasserstoff, C₁-C₈-Alkyl (verzweigt oder unverzweigt) oder CO(OR') (mit R' = C₁-C₈-Alkyl (verzweigt oder unverzweigt)) steht, bedeutet,
R² Wasserstoff, COR^{b}, wobei R^{b} für C₁-C₈-Alkyl(verzweigt oder unverzweigt) steht, bedeutet,
R³ C₁-C₈-Alkyl (verzweigt oder unverzweigt), C₃-C₈-Cycloalkyl, Phenyl, substituiertes Phenyl ausgewählt aus der Gruppe 4-Acetamidophenyl, 2-Bromphenyl, 3-Bromphenyl, 4-Bromphenyl, 4-Brom-2-fluorphenyl, 5-Brom-2-fluorphenyl, 3-Brom-4-fluorphenyl, 4-tert. Butylphenyl, 2-Chlor-4-fluorphenyl, 2-Chlor-6-fluorphenyl, 2-Chlorphenyl, 3-Chlorphenyl, 4-Chlorphenyl, 4-Cyanophenyl, 2,3-Dichlorphenyl, 2,4-Dichlorphenyl, 3,4-Dichlorphenyl, 2,3-Dimethoxyphenyl, 3,4-Di-methoxyphenyl, 2,4-Dimethylphenyl, 2,5-Di-methylphenyl, 2-Fluorphenyl, 3-Fluorphenyl, 4-Fluorphenyl, 4-Hexylphenyl, 3-Hydroxy-phenyl, 2-Methoxyphenyl, 2-Methylphenyl, 3-Methylphenyl, 4-Methylphenyl, 4-Nitrophenyl, 3-Phenoxyphenyl, 4-(1-Pyrrolidino)phenyl, 2-(Trifluormethyl)phenyl, 3-(Trifluormethyl)phenyl, 4-(Trifluormethyl)phenyl, 3,4,5-Trimethoxyphenyl, 3-(4-Chlorphenoxy)phenyl und 4-Acetoxy-3-methoxyphenyl, 1-Naphthyl, substituiertes 1-Naphtyl ausgewählt aus der Gruppe 4-Dimethylaminonaphthyl, 2-Ethoxynaphthyl und 4-Methoxynaphthyl, 2-Naphtyl, Chinolin, Anthracen, Phenanthren, Benzothiophen, Benzofurfuryl, Pyrrol, substituiertes Pyrrol ausgewählt aus der Gruppe 2-(1-(Phenylsulfonyl)pyrrol), 2-(N-Methylpyrrol), 2-(N-(3,5-Dichlorphenyl)pyrrol) und 2(1-(4-Chlor-phenyl)pyrrol), 2-Pyridyl, 3-Pyridyl, 4-Pyridyl, Furfuryl, substituiertes Furfuryl ausgewählt aus der Gruppe 2-(5-Acetoxymethylfurfuryl), 2-(5-Methyl-furfuryl), 2-(5-Nitrofurfuryl), 2-[5-(3-Nitrophenyl)furfuryl], 2-[5-(2-Nitro-phenyl)furfuryl], 2-(5-Bromfurfuryl), 2-[5-(4-Chlorphenyl)furfuryl], 2-(4,5-Dimethylfurfuryl), 2-[5-(2-Chlorphenyl]furfuryl], 2- (5-Ethylfurfuryl) und 2-[5-(1,3-Dioxalan)furfuryl], Thiophen, oder substituiertes Thiophen ausgewählt aus der Gruppe 2-(5-Chlorthiophenyl), 2-(5-Methylthiophenyl), 2-(5-Ethylthiophenyl), 2-(3-Methylthiophenyl), 2-(4-Bromthiophenyl), 2-(5-Nitrothiophenyl), 5-(2-Carbonsäurethiophenyl), 2-[4-(Phenylethyl)thiophenyl], 2-[5-(Methylthio)thiophenyl], 2-(3-Bromthiophenyl), 2-(3-Phenoxythiophenyl) und 2-(5-Bromthiophenyl) bedeutet,
X CR⁵, N oder S bedeutet und Y für den Fall, daß X S bedeutet, CR⁶ oder N und in allen anderen Fällen N bedeutet,
R⁴, R⁵ und R⁶ unabhängig voneinander Wasserstoff, C₁-C₈-Alkyl (verzweigt oder unverzweigt) oder CH₂CO(OR'), wobei R' jeweils die oben angegebene Bedeutung hat oder auch Wasserstoff bedeutet, und deren pharmazeutisch akzeptable Salze ausgenommen Verbindungen, bei denen entweder gleichzeitig R¹C(CH₃)₃, R² Wasserstoff, R³ unsubstituiertes Phenyl,X S und Y N oder CR⁶ mit R⁶= Wasserstoff oder CH₂-CO₂-Ethyl oder gleichzeitig R¹ C(CH₃)₃, R² Wasserstoff, R³ unsubstituiertes Phenyl, Y NH und X N oder CR⁵ mit R⁵ = CO₂Ethyl bedeutet.

2. Arzneimittel enthaltend als Wirkstoff mindestens ein bicyclisches Imidazo-5-yl-amin der allgemeinen Formel I gemäß Anspruch 1, in der R¹ bis R⁶, X und Y die in Anspruch 1 angegebene Bedeutung haben, in Form der Base oder von pharmazeutisch akzeptablen Salzen.

3. Arzneimittel gemäß Anspruch 2, **dadurch gekennzeichnet, daß** es als Wirkstoff mindestens ein bicyclisches Imidazo-5-yl-amin ausgewählt aus der Gruppe
*tert*-Butyl-(5-furan-2-yl-imidazo[1,2-b][1,2,4]triazol-6-yl)-amin,
*tert*-Butyl-(6-furan-2-yl-imidazo[2,1-b]thiazol-5-yl)-amin, (5-*tert*-Butylamino-6-furan-2-yl-imidazo[2,1-b]thiazol-3-yl)-essigsäure,
*tert*-Butyl-(5-pyridin-2-yl-imidazo[1,2-b] [1,2,4]triazol-6-yl)-amin,
*tert*-Butyl-(6-pyridin-2-yl-imidazo[2,1-b]thiazol-5-yl)-amin,
*tert*-Butyl-(5-pyridin-3-yl-imidazo[1,2-b] [1,2,4]triazol-6-yl)-amin,
*tert*-Butyl-(5-pyridin-4-yl-imidazo[1,2-b] [1,2,4]triazol-6-yl)-amin,
*tert*-Butyl-(6-cyclohexyl-imidazo[2,1-b]thiazol-5-yl)-amin, *tert*-Butyl-(5-methyl-imidazo[1,2-b][1,2,4]triazol-6-yl)-amin,
*tert*-Butyl-(6-methyl-imidazo[2,1-b]thiazol-5-yl)-amin,
Cyclohexyl-(5-pyridin-2-yl-imidazo[1,2-b] [1,2,4]triazol-6-yl)-amin,
Cyclohexyl-(6-pyridin-2-yl-imidazo[2,1-b]thiazol-5-yl)-amin,
(5-Cyclohexylamino-6-pyridin-2-yl-imidazo[2,1-b]thiazol-3-yl)-essigsäure,
Cyclohexyl-(6-pyridin-4-yl-imidazo[2,1-b]thiazol-5-yl)-amin,
Cyclohexyl-(6-cyclohexyl-imidazo[2,1-b]thiazol-5-yl)-amin, (6-Cyclohexyl-5-cyclohexylamino-imidazo[2,1-b]thiazol-3-yl)-essigsäure,
(5-Cyclohexylamino-6-methyl-imidazo[2,1-b]thiazol-3-yl)-essig-säure,
6-Cyclohexyl-imidazo[2,1-b]thiazol-5-ylamino)-essigsäuremethylester,
(6-Methyl-imidazo[2,1-b]thiazol-5-ylamino)-essigsäuremethylester,
*tert*-Butyl-(2-phenyl-5H-imidazo[1,2-b]pyrazol-3-yl)-amin,
3-(5-*tert*-Butylamino-imidazo[2,1-b]thiazol-6-yl)-phenol, *tert*-Buty1-[6-(3,4-dimethoxy-phenyl)-imidazo[2,1-b]thiazol-5-yl]-amin,
*tert*-Butyl-[5-(2,3-dichlor-phenyl) -imidazo[1,2-b] [1,2,4]triazol-6-yl]-amin,
*tert*-Butyl-[6-(2,3-dichlor-phenyl)-imidazo[2,1-b]thiazol-5-yl]-amin,
*tert*-Butyl-[5-(2,4-dichlor-phenyl) -imidazo[1,2-b] [1,2,4]triazol-6-yl]-amin,
*tert*-Butyl-[6-(2,4-dichlor-phenyl)-imidazo[2,1-b]thiazol-5-yl]-amin,
*tert*-Butyl-[5-(2-methoxy-phenyl) -imidazo[1,2-b] [1,2,4]triazol-6-yl]-amin,
*tert*-Butyl-[6-(2-methoxy-phenyl)-imidazo[2,1-b]thiazol-5-yl]-amin,
[5-*tert*-Butylamino-6-(2-methoxy-phenyl)-imidazo[2,1-b] thiazol-3-yl]-essigsäure,
tert-Butyl-(5-o-tolyl-imidazo[1,2-b][1,2,4]triazol-6-yl)-amin,
*tert*-Butyl-(6-o-tolyl-imidazo[2,1-b]thiazol-5-yl)-amin,
*tert*-Butyl-[5-(2,3-dimethoxy-phenyl) -imidazo[1,2-b][1,2,4]triazol-6-yl]-amin,
*tert*-Butyl-[6-(2,3-dimethoxy-phenyl)-imidazo[2,1-b] thiazol-5-yl]-amin,
*tert*-Butyl-(6-p-tolyl-imidazo[2,1-b]thiazol-5-yl)-amin,
(5-*tert*-Butylamino-6-methyl-imidazo[2,1-b]thiazol-3-yl)-essigsäure,
N-*tert*-Butyl-N-(6-phenyl-imidazo[2,1-b]thiazol-5-yl)-acetamid,
N-*tert*-Butyl-N-(6-o-tolyl-imidazo[2,1-b]thiazol-5-yl)-acetamid,
Butyl-[6-(4-*tert*-butyl-phenyl)-2-methyl-imidazo[2,1-b]thiazol-5-yl]-amin,
*tert*-Butyl-[5-(2-fluorphenyl)-imidazo[1,2-b][1,2,4]triazol-6-yl]-amin,
*tert*-Butyl-[6-(2-fluorphenyl)-imidazo[2,1-b]thiazol-5-yl]-amin,
*tert*-Butyl-(5-naphthalen-1-yl-imidazo[1,2-b][1,2,4]triazol-6-yl)-amin,
Cyclohexyl-(5-naphthalin-1-yl-imidazo[1,2-b][1,2,4]triazol-6-yl)-amin,
[5-(2-Bromphenyl)-imidazo[1,2-b][1,2,4]triazol-6-yl]-(1,1,3,3-tetramethyl-butyl)-amin,
N-[4-(6-Cyclohexylamino-imidazo[1,2-b][1,2,4]triazol-5-yl)-phenyl]-acetamid,
*tert*-Butyl-[5-(2,5-dimethyl-phenyl)-imidazo[1,2-b][1,2,4]triazol-6-yl]-amin,
Cyclohexyl-[6-(2,4-dimethyl-phenyl)-imidazo[2,1-b]thiazol-5-yl]-amin,
Cyclohexyl-[6-(2,5-dimethylphenyl)-imidazo[2,1-b]thiazol-5-yl]-amin,
N-*tert*-Butyl-N-(6-p-tolyl-imidazo[2,1-b]thiazol-5-yl)-acetamid,
[5-(2,4-Dimethyl-phenyl) -imidazo[1,2-b] [1,2,4]triazol-6-yl]-(1,1,3,3-tetramethyl-butyl)-amin,
[5-(2,5-Dimethyl-phenyl)-imidazo[1,2-b][1,2,4]triazol-6-yl]-(1,1,3,3-tetramethyl-butyl)-amin,
N-Butyl-N-[5-(2-chlor-6-fluorphenyl) -imidazo[1,2-b][1,2,4]triazol-6-yl]-acetamid,
N-Butyl-N-[6-(4-tert-butyl-phenyl)-2-methyl-imidazo[2,1-b]thiazol-5-yl]-acetamid
oder der pharmazeutisch akzeptablen Salze dieser Verbindungen enthält.

4. Verwendung von mindestens einem bicyclischen Imidazo-5-yl-amin gemäß Anspruch 1 zusammen mit einem oder mehreren Hilfsstoffen zur Herstellung eines Arzneimittels zur Bekämpfung von Schmerz.

5. Verfahren zur Herstellung von bicyclischen Imidazo-5-yl-aminen gemäß Anspruch 1 durch Dreikomponentenreaktion aus Amidin, Aldehyd und Isonitril, **dadurch gekennzeichnet, daß** die Synthese der Verbindungen in Dichlormethan als Lösungsmittel und in Gegenwart von Perchlorsäure erfolgt, wobei die Ausgangsverbindungen nacheinander in der Reihenfolge Amidin, Aldehyd und Isonitril zugegeben werden und die entstehenden Produkte gegebenenfalls anschließend mit einer Verbindung R²Hal umgesetzt werden.

## Claims

1. Bicyclic imidazo-5-yl-amines of the general formulae (I) 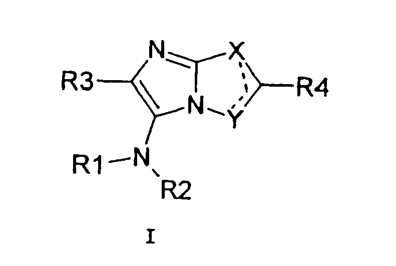 wherein
R¹ denotes C(CH₃)₃, phenyl, C₄-C₈-cycloalkyl, 1,1,3,3-tetramethylbutyl or CH₂R^{a}, wherein R^{a} represents hydrogen, C₁-C₈-alkyl (branched or unbranched) or CO(OR') (where R' = C₁-C₈-alkyl (branched or unbranched)),
R² denotes hydrogen, COR^{b}, wherein R^{b} represents C₁-C₈-alkyl (branched or unbranched),
R³ denotes C₁-C₈-alkyl (branched or unbranched), C₃-C₈-cycloalkyl, phenyl, substituted phenyl chosen from the group consisting of 4-acetamidophenyl, 2-bromophenyl, 3-bromophenyl, 4-bromophenyl, 4-bromo-2-fluorophenyl, 5-bromo-2-fluorophenyl, 3-bromo-4-fluorophenyl, 4-tert-butylphenyl, 2-chloro-4-fluorophenyl, 2-chloro-6-fluorophenyl, 2-chlorophenyl, 3-chlorophenyl, 4-chlorophenyl, 4-cyanophenyl, 2,3-dichlorophenyl, 2,4-dichlorophenyl, 3,4-dichlorophenyl, 2,3-dimethoxyphenyl, 3,4-dimethoxyphenyl, 2,4-dimethylphenyl, 2,5-dimethylphenyl, 2-fluorophenyl, 3-fluorophenyl, 4-fluorophenyl, 4-hexylphenyl, 3-hydroxyphenyl, 2-methoxyphenyl, 2-methylphenyl, 3-methylphenyl, 4-methylphenyl, 4-nitrophenyl, 3-phenoxyphenyl, 4-(1-pyrrolidino)phenyl, 2-(trifluoromethyl)phenyl, 3-(trifluoromethyl)phenyl, 4-(trifluoromethyl)phenyl, 3,4,5-trimethoxyphenyl, 3-(4-chlorophenoxy)phenyl and 4-acetoxy-3-methoxyphenyl, 1-naphthyl, substituted 1-naphthyl chosen from the group consisting of 4-dimethylaminonaphthyl, 2-ethoxynaphthyl and 4-methoxynaphthyl, 2-naphthyl, quinoline, anthracene, phenanthrene, benzothiophene, benzofurfuryl, pyrrole, substituted pyrrole chosen from the group consisting of 2-(1-(phenylsulfonyl)pyrrole), 2-(N-methylpyrrole), 2-(N-(3,5-dichlorophenyl)pyrrole and 2-(1-(4-chlorophenyl)pyrrole), 2-pyridyl, 3-pyridyl, 4-pyridyl, furfuryl, substituted furfuryl chosen from the group consisting of 2-(5-acetoxymethylfurfuryl), 2-(5-methylfurfuryl), 2-(5-nitrofurfuryl), 2-[5-(3-nitrophenyl)furfuryl], 2-[5-(2-nitrophenyl)furfuryl], 2-(5-bromofurfuryl), 2-[5-(4-chlorophenyl)furfuryl], 2-(4,5-dimethylfurfuryl), 2-[5-(2-chlorophenyl)furfuryl], 2-(5-ethylfurfuryl) and 2-[5-(1,3-dioxalane)furfuryl], thiophene, or substituted thiophene chosen from the group consisting of 2-(5-chlorothiophenyl), 2-(5-methylthiophenyl), 2-(5-ethylthiophenyl), 2-(3-methylthiophenyl), 2-(4-bromothiophenyl), 2-(5-nitrothiophenyl), 5-(2-carboxythiophenyl), 2-[4-(phenylethyl)thiophenyl], 2-[5-(methylthio)thiophenyl], 2-(3-bromothiophenyl), 2-(3-phenoxythiophenyl) and 2-(5-bromothiophenyl),
X denotes CR⁵, N or S and Y, in the case where X denotes S, denotes CR⁶ or N and in all other cases denotes N,
R⁴, R⁵ and R⁶ independently of one another hydrogen, C₁-C₈-alkyl (branched or unbranched) or CH₂CO(OR'), wherein R' in each case has the abovementioned meaning or also denotes hydrogen, and pharmaceutically acceptable salts thereof
excluding compounds in which either at the same time R¹ denotes C(CH₃)₃, R² denotes hydrogen, R³ denotes unsubstituted phenyl, X denotes S and Y denotes N or CR⁶, where R⁶ = hydrogen or CH₂-CO₂-ethyl, or at the same time R¹ denotes C(CH₃)₃, R² denotes hydrogen, R³ denotes unsubstituted phenyl, Y denotes NH and X denotes N or CR⁵, where R⁵ = CO₂ethyl.

2. Medicament comprising as the active compound at least one bicyclic imidazo-5-yl-amine of the general formula I according to claim 1, in which R¹ to R⁶, X and Y have the meaning given in claim 1, in the form of the base or of pharmaceutically acceptable salts.

3. Medicament according to claim 2, **characterized in that** it comprises as the active compound at least one bicyclic imidazo-5-yl-amine chosen from the group consisting of
*tert*-butyl-(5-furan-2-yl-imidazo[1,2-b][1,2,4]triazol-6-yl)-amine,
*tert*-butyl-(6-furan-2-yl-imidazo[2,1-b]thiazol-5-yl)-amine,
(5-*tert*-butylamino-6-furan-2-yl-imidazo[2,1-b]thiazol-3-yl)-acetic acid,
*tert*-butyl-(5-pyridin-2-yl-imidazo[1,2-b][1,2,4]triazol-6-yl)-amine,
*tert*-butyl-(6-pyridin-2-yl-imidazo[2,1-b]thiazol-5-yl)-amine,
*tert*-butyl-(5-pyridin-3-yl-imidazo[1,2-b][1,2,4]triazol-6-yl)-amine,
tert-butyl-(5-pyridin-4-yl-imidazo[1,2-b][1,2,4]triazol-6-yl)-amine,
*tert*-butyl-(6-cyclohexyl-imidazo[2,1-b]thiazol-5-yl)-amine,
*tert*-butyl-(5-methyl-imidazo[1,2-b][1,2,4]triazol-6-yl)-amine,
*tert*-butyl-(6-methyl-imidazo[2,1-b]thiazol-5-yl)-amine,
cyclohexyl-(5-pyridin-2-yl-imidazo[1,2-b][1,2,4]triazol-6-yl)-amine,
cyclohexyl-(6-pyridin-2-yl-imidazo[2,1-b]thiazol-5-yl)-amine,
(5-cyclohexylamino-6-pyridin-2-yl-imidazo[2,1-b]thiazol-3-yl)-acetic acid,
cyclohexyl-(6-pyridin-4-yl-imidazo[2,1-b]thiazol-5-yl)-amine,
cyclohexyl-(6-cyclohexyl-imidazo[2,1-b]thiazol-5-yl)-amine,
(6-cyclohexyl-5-cyclohexylamino-imidazo[2,1-b]thiazol-3-yl)-acetic acid,
(5-cyclohexylamino-6-methyl-imidazo[2,1-b]thiazol-3-yl)-acetic acid,
methyl (6-cyclohexyl-imidazo[2,1-b]thiazol-5-ylamino)-acetate,
methyl (6-methyl-imidazo[2,1-b]thiazol-5-ylamino)-acetate,
*tert*-butyl-(2-phenyl-5H-imidazo[1,2-b]pyrazol-3-yl)-amine,
3-(5-*tert*-butylamino-imidazo[2,1-b]thiazol-6-yl)-phenol,
*tert*-butyl-[6-(3,4-dimethoxy-phenyl)-imidazo[2,1-b]thiazol-5-yl]-amine,
*tert*-butyl-[5-(2,3-dichloro-phenyl)-imidazo[1,2-b][1,2,4]triazol-6-yl]-amine,
*tert*-butyl-[6-(2,3-dichloro-phenyl)-imidazo[2,1-b]thiazol-5-yl]-amine,
*tert*-butyl-[5-(2,4-dichloro-phenyl)-imidazo[1,2-b][1,2,4]triazol-6-yl]-amine,
*tert*-butyl-[6-(2,4-dichloro-phenyl)-imidazo[2,1-b]thiazol-5-yl]-amine,
*tert*-butyl-[5-(2-methoxy-phenyl)-imidazo[1,2-b][1,2,4]triazol-6-yl]-amine,
*tert*-butyl-[6-(2-methoxy-phenyl)-imidazo[2,1-b]thiazol-5-yl]-amine,
[5-*tert*-butylamino-6-(2-methoxy-phenyl)-imidazo[2,1-b]thiazol-3-yl]-acetic acid,
*tert*-butyl-(5-o-tolyl-imidazo[1,2-b][1,2,4]triazol-6-yl)-amine,
*tert*-butyl-(6-o-tolyl-imidazo[2,1-b]thiazol-5-yl)-amine,
*tert*-butyl-[5-(2,3-dimethoxy-phenyl)-imidazo[1,2-b][1,2,4]triazol-6-yl]-amine,
*tert*-butyl-[6-(2,3-dimethoxy-phenyl)-imidazo[2,1-b]thiazol-5-yl]-amine,
*tert*-butyl-(6-p-tolyl-imidazo[2,1-b]thiazol-5-yl)-amine,
(5-*tert*-butylamino-6-methyl-imidazo[2,1-b]thiazol-3-yl)-acetic acid,
N-*tert*-butyl-N-(6-phenyl-imidazo[2,1-b]thiazol-5-yl)-acetamide,
N-*tert*-butyl-N-(6-o-tolyl-imidazo[2,1-b]thiazol-5-yl)-acetamide,
butyl-[6-(4-tert-butyl-phenyl)-2-methyl-imidazo[2,1-b]thiazol-5-yl]amine,
*tert*-butyl-[5-(2-fluorophenyl)-imidazo[1,2-b][1,2,4triazol-6-yl]-amine,
*tert*-butyl-[6-(2-fluorophenyl)-imidazo[2,1-b]thiazol-5-yl]-amine,
*tert*-butyl-(5-naphthalen-1-yl-imidazo[1,2-b][1,2,4]triazol-6-yl)-amine,
cyclohexyl-(5-naphthalen-1-yl-imidazo[1,2-b][1,2,4]triazol-6-yl)-amine,
[5-(2-bromophenyl)-imidazo[1,2-b][1,2,4]triazol-6-yl]-(1,1,3,3-tetramethyl-butyl)-amine,
N-[4-(6-cyclohexylamino-imidazo[1,2-b][1,2,4]triazol-5-yl)-phenyl)-acetamide,
*tert*-butyl-[5-(2,5-dimethyl-phenyl)-imidazo[1,2-b][1,2,4]triazol-6-yl]-amine,
cyclohexyl-[6-(2,4-dimethyl-phenyl)-imidazo[2,1-b]thiazol-5-yl]-amine,
cyclohexyl-[6-(2,5-dimethylphenyl)-imidazo[2,1-b]thiazol-5-yl]-amine,
N-*tert*-butyl-N-(6-p-tolyl-imidazo[2,1-b]thiazol-5-yl)-acetamide,
[5-(2,4-dimethyl-phenyl)-imidazo[1,2-b][1,2,4]triazol-6-yl]-(1,1,3,3-tetramethyl-butyl)-amine,
[5-(2,5-dimethyl-phenyl)-imidazo[1,2-b][1,2,4]triazol-6-yl]-(1,1,3,3-tetramethyl-butyl)-amine,
N-butyl-N-[5-(2-chloro-6-fluorophenyl)-imidazo[1,2-b][1,2,4]triazol-6-yl]-acetamide and
N-butyl-N-[6-(4-*tert*-butyl-phenyl)-2-methylimidazo[2,1-b]thiazol-5-yl]-acetamide
or the pharmaceutically acceptable salts of these compounds.

4. Use of at least one bicyclic imidazo-5-yl-amine according to claim 1 together with one or more auxiliary substances for the preparation of a medicament for combating pain.

5. Process for the preparation of bicyclic imidazo-5-yl-amines according to claim 1 by a three-component reaction from amidine, aldehyde and isonitrile, **characterized in that** the synthesis of the compounds is carried out in methylene chloride as the solvent and in the presence of perchloric acid, the starting compounds being added in succession in the sequence amidine, aldehyde and isonitrile and the products formed optionally then being reacted with a compound R²Hal.

## Revendications

1. Imidazo-5-yl-amines bicycliques de formule générale I 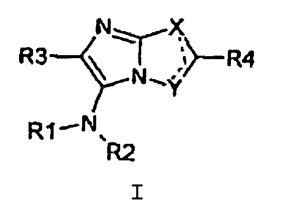 dans laquelle .
R¹ est un reste C(CH₃)₃, phényle, alkyle en C₄ à C₈, 1,1,3,3-tétraméthylbutyle ou CH₂R^{a}, où R^{a} représente l'hydrogène, un radical alkyle en C₁ à C₈ (ramifié ou non ramifié) ou un reste CO(OR') (dans lequel R' est un radical alkyle en C₁ à C₈ (ramifié ou non ramifié)),
R² représente l'hydrogène, un reste COR^{b}, dans lequel R^{b} est un radical alkyle en C₁ à C₈ (ramifié ou non ramifié),
R³ représente un reste alkyle en C₁ à C₈ (ramifié ou non ramifié), cycloalkyle en C₃ à C₈, phényle, phényle substitué choisi dans le groupe des restes 4-acétamidophényle, 2-bromophényle, 3-bromophényle, 4-bromophényle, 4-bromo-2-fluorophényle, 5-bromo-2-fluorophényle, 3-bromo-4-fluorophényle, 4-tertiobutylphényle, 2-chloro-4-fluorophényle, 2-chloro-6-fluorophényle, 2-chlorophényle, 3-chlorophényle, 4-chlorophényle, 4-cyanophénylé, 2,3-dichlorophényle, 2,4-dichlorophényle, 3,4-dichlorophényle, 2,3-diméthoxyphényle, 3,4-diméthoxyphényle, 2,4-diméthylphényle, 2,5-diméthylphényle, 2-fluorophényle, 3-fluorophényle, 4-fluorophényle, 4-hexylphényle, 3-hydroxyphényle, 2-méthoxyphényle, 2-méthylphényle, 3-méthylphényle, 4-méthylphényle, 4-nitrophényle, 3-phénoxyphényle, 4-(1-pyrrolidino)phényle, 2-(trifluorométhyl)phényle, 3-(trifluorométhyl)phényle, 4-(trifluorométhyl)phényle, 3,4,5-triméthoxyphényle, 3-(4-chlorophénoxy)phényle et 4-acétoxy-3-méthoxyphényle, 1-naphtyle, 1-naphtyle substitué choisi dans le groupe des restes 4-diméthylaminonaphtyle, 2-éthoxynaphtyle et 4-méthoxynaphtyle, 2-naphtyle, quinoléine, anthracène, phénanthrène, benzothiophène, benzofurfuryle, pyrrole, pyrrole substitué choisi dans le groupe des restes 2-(1-(phénylsulfonyl)pyrrole), 2-(N-méthylpyrrole), 2-(N-(3,5-dichlorophényl)pyrrole) et 2-(1-(4-chlorophényl)pyrrole), 2-pyridyle, 3-pyridyle, 4-pyridyle, furfuryle, furfuryle substitué choisi dans le groupe des restes 2-(5-acétoxyméthylfurfuryle), 2-(5-méthylfurfuryle), 2-(5-nitrofurfuryle), 2-[5-(3-nitrophényl)furfuryle], 2-[5-(2-nitrophényl)furfuryle], 2-(5-bromofurfuryle), 2-[5-(4-chlorophényl)furfuryle], 2-(4,5-diméthylfurfuryle), 2-[5-(2-chlorophényl)furfuryle], 2-(5-éthylfurfuryle) et 2-[5-(1,3-dioxalanne)furfuryle], thiophène ou thiophène substitué choisi dans le groupe des restes 2-(5-chlorothiophényle), 2-(5-méthylthiophényle), 2-(5-éthylthiophényle), 2-(3-méthylthiophényle), 2-(4-bromothiophényle), 2-(5-nitrothiophényle), 5-(2-carboxythiophényle), 2-[4-(phényléthyl)thiophényle], 2-[5-(méthylthio)thiophényle], 2-(3-bromothiophényle), 2-(3-phénoxythiophényle) et 2-(5-bromothiophényle),
X représente CR⁵, N ou S et Y représente CR⁶ ou N, au cas où X désigne S, et représente N dans tous les autres cas,
R⁴, R⁵ et R⁶ représentent, indépendamment les uns des autres, l'hydrogène, un reste alkyle en C₁ à C₈ (ramifié ou non ramifié) ou un groupe CH₂CO(OR'), dans lequel R' a dans chaque cas la définition indiquée ci-dessus ou désigne aussi l'hydrogène,
et leurs sels pharmaceutiquement acceptables,
excepté les composés dans lesquels, en même temps, R¹ représente C(CH₃)₃, R² représente l'hydrogène, R³ est un reste phényle non substitué, X représente S et Y représente N ou un groupe CR⁶, dans lequel R⁶ est l'hydrogène ou un radical CH₂-CO₂-éthyle, ou dans lesquels, en même temps, R¹ représente un reste C(CH₃)₃, R² représente l'hydrogène, R³ est un reste phényle non substitué, Y représente NH et X représente N ou un groupe CR⁵ dans lequel R⁵ est un radical CO₂éthyle.

2. Médicament contenant, comme substance active, au moins une imidazo-5-yl-amine bicyclique de formule générale I suivant la revendication 1, dans lequel R¹ à R⁶, X et Y ont la définition indiquée dans la revendication 1, sous forme de la base ou de sels pharmaceutiquement acceptables.

3. Médicament suivant la revendication 2, **caractérisé en ce qu'**il contient, comme substance active, au moins une imidazo-5-yl-amine bicyclique choisie dans le groupe des composés :
tertiobutyl-(5-furanne-2-yl-imidazo[1,2-b][1,2,4]triazole-6-yl)-amine,
tertiobutyl-(6-furanne-2-yl-imidazo[2,1-b]thiazole-5-yl)-amine,
acide (5-tertiobutylamino-6-furanne-2-yl-imidazo[2,1-b]-thiazole-3-yl)-acétique,
tertiobutyl-(5-pyridine-2-yl-imidazo[1,2-b][1,2,4]triazole-6-yl)-amine,
tertiobutyl-(6-pyridine-2-yl-imidazo[2,1-b]thiazole-5-yl)-amine,
tertiobutyl-(5-pyridine-3-yl-imidazo[1,2-b][1,2,4]triazole-6-yl)-amine,
tertiobutyl-(5-pyridine-4-yl-imidazo[1,2-b][1,2,4]triazole-6-yl)-amine,
tertiobutyl-(6-cyclohexyl-imidazo[2,1-b]thiazole-5-yl)-amine,
tertiobutyl-(5-méthyl-imidazo[1,2-b][1,2,4]triazole-6-yl)-amine,
tertiobutyl-(6-méthyl-imidazo[2,1-b]thiazole-5-yl)-amine, cyclohexyl-(5-pyridine-2-yl-imidazo[1,2-b][1,2,4]triazole-6-yl)-amine,
cyclohexyl-(6-pyridine-2-yl-imidazo[2,1-b]thiazole-5-yl)-amine,
acide (5-cyclohexylamino-6-pyridine-2-yl-imidazo[2,1-b]-thiazole-3-yl)-acétique,
cyclohexyl-(6-pyridine-4-yl-imidazo[2,1-b]thiazole-5-yl)-amine,
cyclohexyl-(6-cyclohexyl-imidazo[2,1-b]thiazole-5-yl)-amine,
acide (6-cyclohexyl-5-cyclohexylamino-imidazo[2,1-b]-thiazole-3-yl)-acétique,
acide (5-cyclohexylamino-6-méthyl-imidazo[2,1-b]thiazole-3-yl)-acétique,
ester méthylique d'acide (6-cyclohexyl-imidazo[2,1-b]-thiazole-5-ylamino)-acétique,
ester méthylique d'acide (6-méthyl-imidazo[2,1-b]thiazole-5-ylamino)-acétique,
tertiobutyl-(2-phényl-5H-imidazo[1,2-b]pyrazole-3-yl)-amine,
3-(5-tertiobutylamino-imidazo[2,1-b]thiazole-6-yl)-phénol,
tertiobutyl-[6-(3,4-diméthoxy-phényl)-imidazo[2,1-b]-thiazole-5-yl]-amine,
tertiobutyl-[5-(2,3-dichloro-phényl)-imidazo[1,2-b][1,2,4]-triazole-6-yl]-amine,
tertiobutyl-[6-(2,3-dichloro-phényl)-imidazo[2,1-b]-thiazole-5-yl]-amine,
tertiobutyl-[5-(2,4-dichloro-phényl)-imidazo[1,2-b][1,2,4]-triazole-6-yl]-amine,
tertiobutyl-[6-(2,4-dichloro-phényl)-imidazo[2,1-b]-thiazole-5-yl]-amine,
tertiobutyl-[5-(2-méthoxy-phényl)-imidazo[1,2-b][1,2,4]-triazole-6-yl]-amine,
tertiobutyl-[6-(2-méthoxy-phényl)-imidazo[2,1-b]thiazole-5-yl]-amine,
acide [5-tertiobutylamino-6-(2-méthoxy-phényl)-imidazo-[2,1-b]thiazole-3-yl]-acétique,
tertiobutyl-(5-o-tolyl-imidazo[1,2-b][1,2,4]triazole-6-yl)-amine,
tertiobutyl-(6-o-tolyl-imidazo[2,1-b]thiazole-5-yl)-amine,
tertiobutyl-[5-(2,3-diméthoxy-phényl)-imidazo[1,2-b]-[1,2,4]triazole-6-yl]-amine,
tertiobutyl-[6-(2,3-diméthoxy-phényl)-imidazo[2,1-b]-thiazole-5-yl]-amine,
tertiobutyl-(6-p-tolyl-imidazo[2,1-b]thiazole-5-yl)-amine,
acide (5-tertiobutylamino-6-méthyl-imidazo[2,1-b]thiazole-3-yl)-acétique,
N-tertiobutyl-N-(6-phényl-imidazo[2,1-b]thiazole-5-yl)-acétamide,
N-tertiobutyl-N-(6-o-tolyl-imidazo[2,1-b]thiazole-5-yl)-acétamide,
butyl-[6-(4-tertiobutyl-phényl)-2-méthyl-imidazo[2,1-b]-thiazole-5-yl]-amine,
tertiobutyl-[5-(2-fluorophényl)-imidazo[1,2-b][1,2,4]-triazole-6-yl]-amine,
tertiobutyl-[6-(2-fluorophényl)-imidazo[2,1-b]thiazole-5-yl]-amine,
tertiobutyl-(5-naphtalène-1-yl-imidazo[1,2-b][1,2,4]-triazole-6-yl)-amine,
cyclohexyl-(5-naphtalène-1-yl-imidazo[1,2-b][1,2,4]-triazole-6-yl)-amine,
[5-(2-bromophényl)-imidazo[1,2-b][1,2,4]triazole-6-yl]-(1,1,3,3-tétraméthyl-butyl)-amine,
N-[4-(6-cyclohexylamino-imidazo[1,2-b][1,2,4]triazole-5-yl)-phényl]-acétamide,
tertiobutyl-[5-(2,5-diméthyl-phényl)-imidazo[1,2-b][1,2,4]-triazole-6-yl]-amine,
cyclohexyl-[6-(2,4-diméthyl-phényl)-imidazo[2,1-b]thiazole-5-yl]-amine,
cyclohexyl-[6-(2,5-diméthylphényl)-imidazo[2,1-b]thiazole-5-yl]-amine,
N-tertiobutyl-N-(6-p-tolyl-imidazo[2,1-b]thiazole-5-yl)-acétamide,
[5-(2,4-diméthyl-phényl)-imidazo[1,2-b][1,2,4]triazole-6-yl]-(1,1,3,3-tétraméthyl-butyl)-amine,
[5-(2,5-diméthyl-phényl)-imidazo[1,2-b][1,2,4]triazole-6-yl]-(1,1,3,3-tétraméthyl-butyl)-amine,
N-butyl-N-[5-(2-chloro-6-fluorophényl)-imidazo[1,2-b]-[1,2,4]triazole-6-yl]-acétamide et
N-butyl-N-[6-(4-tertiobutyl-phényl)-2-méthyl-imidazo-[2,1-b]thiazole-5-yl]-acétamide,
ou de sels pharmaceutiquement acceptables de ces composés.

4. Utilisation d'au moins une imidazo-5-yl-amine bicyclique suivant la revendication 1 conjointement avec une ou plusieurs substances auxiliaires pour la préparation d'un médicament destiné à combattre la douleur.

5. Procédé de préparation d'imidazo-5-yl-amine bicyclique suivant la revendication 1 par réaction de trois composants formés d'amidine, aldéhyde et isonitrile, **caractérisé en ce que** la synthèse des composés est conduite dans du dichlorométhane utilisé comme solvant et en présence d'acide perchlorique, les composés de départ étant ajoutés successivement dans l'ordre amidine, aldéhyde et isonitrile et les produits formés étant ensuite éventuellement amenés à réagir avec un composé de formule R²Hal.
